# EUROPEAN PATENT SPECIFICATION

(11) **EP 1 444 197 B1**
(45) Date of publication and mention of the grant of the patent: **30.03.2005**
(21) Application number: 02798312.1
(22) Date of filing: 15.11.2002
(51) Int. Cl.: C07C 235/34, C07C 323/52, C07D 307/79, C07D 317/60, C07D 319/18, C07D 333/24, C07D 333/28, C07D 307/54, C07D 261/08, C07D 333/60, C07D 277/64, C07D 213/56, C07D 239/26, C07D 215/12, C07D 241/42, A01N 37/18, A01N 43/00

(54) **NOVEL PHENYL-PROPARGYLETHER DERIVATIVES**
NEUE PHENYL-PROPARGYLETHER DERIVATE
NOUVEAUX DERIVES DE PHENYL-PROPARGYLETHER

(30) Priority: 16.11.2001 GB 0127554
(43) Date of publication of application: 11.08.2004
(73) Proprietor: Syngenta Participations AG, 4058 Basel (CH)
(72) Inventor: ZELLER, Martin, Syngenta Crop Prot. Muenchwilen AG, CH-4333 Muenchwilwn (CH); LAMBERTH, Clemens, Syngenta Crop Protection AG, CH-4058 Basel (CH)
(74) Representative: Weichsel, Christian Henner
(86) International application number: PCT/EP2002/012848
(87) International publication number: WO 2003/041728

(56) References cited:
- WO-A-00/41998
- WO-A-01/87822
- WO-A-96/17840

## Description

The present invention relates to novel enantiomeric forms of phenyl-propargylether derivatives of formula I below. It relates to the preparation of those enantiomeric substances and to agrochemical compositions comprising at least one of the compounds of formula I as active ingredient. The invention relates also to the preparation of the said compositions and to the use of the compounds or of the compositions in controlling or preventing the infestation of plants by phytopathogenic microorganisms, especially fungi.

The invention relates to phenyl-propargylether derivatives of the general formula I 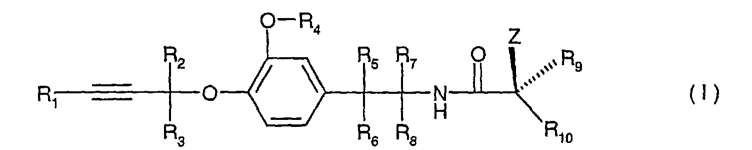 including the optical isomers thereof and mixtures of such isomers, wherein
R₁ is hydrogen, alkyl, cycloalkyl or optionally substituted aryl,
R₂ and R₃ are each independently hydrogen or alkyl,
R₄ is alkyl, alkenyl or alkynyl,
R₅, R₆, R₇, and R₈ are each independently hydrogen or alkyl,
R₉ is hydrogen, optionally substituted alkyl, optionally substituted alkenyl or optionally substituted alkynyl,
R₁₀ is optionally substituted aryl or optionally substituted heteroaryl, and
Z is optionally substituted aryloxy, optionally substituted alkoxy, optionally substituted alkenyloxy or optionally substituted alkynyloxy

The compounds of formula I is understood to encompass the pure enantiomer of the displayed formula I as well as substantially enantio-enriched forms, as usually obtained from large scale synthesis processes. The asymmetrical carbon atom to which the enantiomeric form of present invention relates is the alpha-carbon atom in the carboxylic acid, forming the amide function in the compounds of formula I. This carbon atom is marked in formula I with the indication of the absolute configuration according to the Cahn-Ingold-Prelog rules. For example, where R₉ is hydrogen, R₁₀ is the optionally substituted phenyl moiety, and Z is any defined radical, the asymmetrically substituted carbon atom is in the (S)-configuration.

The enantio-enriched forms according to present invention contain 70% or more of the pure enantiomer of formula I, while the difference to 100% of the compound of formula I is the mirror-image enantiomer. In contrast to present invention, the formerly known compounds of formula have been obtained in racemic forms, with in general comprised about equal amounts of the two enantiomer forms. In preferred embodiments of this invention the amount of the enantiomer in the compounds of formula I is above 80%, more preferred above 90%, especially above 95%, for example 97%, 98% or 99%.

The presence of more asymmetrical carbon atoms in the compounds of formula I , in addition to the alpha-carbon atom of the carboxylic, means that the enantiomers of compounds of formula I can occur in stereoisomeric forms, i.e. as several diastereomers. As a result of the presence of a possible aliphatic C=C double bond, also geometric isomerism may also occur. Formula I is intended to include all those possible diastereomeric forms of the enantiomer of formula I, and mixtures thereof.

Where the definition of formula I refers to optical isomers, for this document it shall be understood that the optical isomers are only those isomers which result from other possible asymmetrical elements in the molecule, if present, but not to the carbon atom in alpha-position of the amide functional group which is defined with its absolute configuration.

In the above definition aryl includes aromatic hydrocarbon rings like phenyl, naphthyl, anthracenyl, phenanthrenyl and biphenyl like 1,3-biphenyl and 1,4-biphenyl, with phenyl being preferred. The same definition applies where aryl is part of aryloxy or arylthio.
Heteroaryl stands for aromatic ring systems comprising mono-, bi- or tricyclic systems wherein at least one oxygen, nitrogen or sulfur atom is present as a ring member. Examples are furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothiophenyl, benzofuranyl, benzimidazolyl, indazolyl, benzotriazolyl, benzothiazolyl, benzoxazolyl, quinolinyl, isoquinolinyl, phthalazinyl, quinoxalinyl, quinazolinyl, cinnolinyl and naphthyridinyl.
The above aryl and heteroaryl groups may be optionally substituted. This means that they may carry one or more identical or different substituents. Normally not more than three substituents are present at the same time. Examples of substituents of aryl or heteroaryl groups are: alkyl, alkenyl, alkynyl, cycloalkyl, cycloalkyl-alkyl, phenyl and phenyl-alkyl, it being possible in turn for all of the preceding groups to carry one or more identical or different halogen atoms; alkoxy; alkenyloxy; alkynyloxy; alkoxyalkyl; haloalkoxy, alkylthio; haloalkylthio; alkylsulfonyl; formyl; alkanoyl; hydroxy; halogen; cyano; nitro; amino; alkylamino; dialkylamino; carboxyl; alkoxycarbonyl; alkenyloxycarbonyl; or alkynyloxycarbonyl. Typical examples include 4-chlorophenyl, 4-bromophenyl, 3,4-dichlorophenyl, 4-chloro-3-fluorophenyl, 3-chloro-4-fluorophenyl, 4-methylphenyl, 4-ethylphenyl, 4-propargyloxyphenyl, 1-naphtyl, 2-naphtyl, 4-biphenylyl, 4'-chloro-4-biphenylyl, 5-chloro-thien2-yl, 5-methyl-thien-2-yl, 5-methyl-fur-2-yl, 5,6,7,8-tetrahydro-1-naphthyl, 5,6,7,8-tetrahydro-2-naphthyl, 3,4dioxomethylenyl-phenyl, 3,4-dioxoethylenyl-phenyl, 6-benzothienyl, 7-benzothienyl, 3-methylphenyl, 4-fluorophenyl, 4-ethenylphenyl, 4-ethynylphenyl, 4-propylphenyl, 4-isopropylphenyl, 4-tert-butylphenyl, 4-ethoxyphenyl, 4-ethynyloxyphenyl, 4-phenoxyphenyl, 4-methylthiophenyl, 4-methylsulfonylphenyl, 4-cyanophenyl, 4-nitrophenyl, 4-methoxycarbonylphenyl, 3-bromophenyl, 3-chlorophenyl, 2-chlorophenyl, 2,4-dichlorophenyl, 3,4,5-trichlorophenyl, 3,4-difluorophenyl, 3,4-dibromophenyl, 3,4-dimethoxyphenyl, 3,4-dimethylphenyl, 3-chloro-4-cyanophenyl, 4-chloro-3-cyanophenyl, 3-bromo-4-methylphenyl, 4-methoxy-3-methylphenyl, 3-fluoro-4-methoxyphenyl, 4-chloro-3-methylphenyl, 4-chloro-3-trifluoromethyl-phenyl, 4-bromo-3-chlorophenyl, 4-trifluoromethylphenyl, 4-trifluoromethoxyphenyl, 4-methoxyphenyl, 4'-methyl-4-biphenylyl, 4'-trifluoromethyl-4-biphenylyl, 4'-bromo-4-biphenylyl, 4'-cyano-4-biphenylyl, 3'4'-dichloro-4-biphenylyl, etc.
Again, the same optional substituent may be present where aryl is part of aryloxy. Optionally substituted alkyl, alkenyl or alkynyl groups may carry one or more substituents selected from halogen, alkyl, alkoxy, alkylthio, cycloalkyl, phenyl, nitro, cyano, hydroxy, mercapto, alkylcarbonyl or alkoxycarbonyl. This also applies where alkyl, alkenyl or alkynyl is part of another substituent like alkoxy, alkylthio, alkylsulfinyl, alkylsulfonyl, alkenylyoxy, alkenylthio, alkenylsulfinyl, alkenylsufonyl, alkynyloxy, alkynylthio, alkynylsulfinyl and alkynylsulfonyl.
Preferably, the number of substituents is no more than three with the exception of halogen, where the alkyl groups may be perhalogenated.
In the above definitions "halogen" includes fluorine, chlorine, bromine and iodine.
The alkyl, alkenyl and alkynyl radicals may be straight-chain or branched. This applies also to the alkyl, alkenyl or alkynyl parts of other alkyl-, alkenyl- or alkynyl-containing groups. Depending upon the number of carbon atoms mentioned, alkyl on its own or as part of another substituent is to be understood as being, for example, methyl, ethyl, propyl, butyl, pentyl, hexyl, heptyl, octyl, nonyl, decyl, undecyl, dodecyl and the isomers thereof, for example isopropyl, isobutyl, tert-butyl or sec-butyl, isopentyl or tert-pentyl.

Cycloalkyl is, depending upon the number of carbon atoms mentioned, cyclopropyl, cyclobutyl, cyclopentyl, cyclohexyl, cycloheptyl or cyclooctyl.
Depending upon the number of carbon atoms mentioned, alkenyl as a group or as a structural element of other groups is to be understood as being, for example -CH=CH₂, -CH₂-CH=CH₂, -CH=CH-CH₃, -CH₂-CH=CH-CH₃, -CH₂-CH₂-CH=CH₂, -CH₂-CH(CH₃)-CH=CH₂, -CH₂-C(CH₃)=CH₂, -CH=CH-(CH₂)₂-CH₃, -CH₂-CH₂-CH=CH-CH₃, -CH₂-CH₂-C(CH₃)=CH-CH₃, -CH(CH₃)-CH₂-CH=CH-CH₃, -CH₂-CH₂-CH=CH-CH₂-CH₃, -CH=CH-(CH₂)₃-CH₃, -CH₂-CH₂-CH=C(CH₃)-CH₃, -CH₂-CH₂-CH=C(CH₃)-CH₂-CH₃, -C(CH₃)=CH₂, -CH(CH₃)-CH=CH₂, -C(CH₃)=CH-CH₃, -CH(CH₃)-CH=CH-CH₃, -CH(CH₃)-CH₂-CH=CH₂, -CH₂-CH(CH₃)-C(CH₃)=CH₂, -CH₂-C(CH₃)=CH-CH₃, -C(CH₃)=CH-(CH₂)₂-CH₃, -CH(CH₃)-CH₂-C(CH₃)=CH-CH₃, -CH(CH₃)-(CH₂)₂-CH=CH₂, -CH(CH₃)-CH₂-CH=CH-CH₂-CH₃, -C(CH₃)=CH-(CH₂)₃-CH₃, -(CH₂)₃-CH=CH₂, -CH(CH₃)-CH₂-C(CH₃)=CH-CH₃, or -CH(CH₃)-CH₂-CH=CH-CH₂-CH₃. Alkynyl as a group or as a structural element of other groups is, for example -C≡CH, -CH₂-C≡CH, -C≡C-CH₃, -CH₂-C≡C-CH₃, -CH₂-CH₂-C≡CH, -C≡C-CH₂-CH₃, -CH₂-CH(CH₃)-C≡CH, -C≡C-(CH₂)₂-CH₃, -CH₂-CH₂-C≡C-CH₃, -CH(CH₃)-CH₂-C≡C-CH₃, -CH₂-CH₂-C≡C-CH₂-CH₃, -C≡C-(CH₂)₃-CH₃, -C≡C-(CH₂)₄-CH₃, -CH(CH₃)-C≡CH, -CH(CH₃)-C≡C-CH₃, -CH(C₂H₅)-C≡C-CH₃, -CH(CH₃)-CH₂-C≡CH, -CH(CH₃)-(CH₂)₂-C≡CH, -CH(CH₃)-CH₂-C≡C-CH₂-CH₃, -(CH₂)₃-C≡CH, or -CH(CH₃)-CH₂-C≡C-CH₂-CH₃ ,depending on the number of carbon atoms present.
A haloalkyl group may contain one or more (identical or different) halogen atoms, and for example may stand for CHCl₂, CH₂F, CCl₃, CH₂Cl, CHF₂, CF₃, CH₂CH₂Br, C₂Cl₅, CH₂Br, CHClBr, CF₃CH₂, etc..

Preferred subgroups of compounds of formula I are those wherein
R₁ is hydrogen, alkyl, cycloalkyl, phenyl or naphthyl; phenyl and naphthyl being optionally substituted by substituents selected from the group comprising alkyl, alkenyl, alkynyl, cycloalkyl, cycloalkyl-alkyl, phenyl and phenylalkyl, where all these groups may in turn be substituted by one or several halogens; alkoxy; alkenyloxy; alkynyloxy; alkoxy-alkyl; haloalkoxy; alkylthio; haloalkylthio; alkylsulfonyl; formyl; alkanoyl; hydroxy; halogen; cyano; nitro; amino; alkylamino; dialkylamino; carboxyl; alkoxycarbonyl; alkenyloxycarbonyl; or alkynyloxycarbonyl; or
R₁ is hydrogen, C₁-C₈alkyl, C₃-C₈cycloalkyl, phenyl or naphthyl; phenyl and naphthyl being optionally substituted by one to three substituents selected from the group comprising C₁-C₈alkyl, C₂-C₈alkenyl, C₂-C₈alkynyl, C₁-C₈haloalkyl, C₁-C₈alkoxy, C₁-C₈haloalkoxy, C₁-C₈alkylthio, C₁-C₈haloalkylthio, C₁-C₈alkylsulfonyl, halogen, cyano, nitro and C₁-C₈alkoxycarbonyl; or
R₁ is hydrogen, C₁-C₈alkyl or phenyl optionally substituted by one to three substituents selected from the group comprising C₁-C₈alkyl, C₁-C₈haloalkyl, C₁-C₈alkoxy, C₁-C₈haloalkoxy, C₁-C₈alkylthio, C₁-C₈haloalkylthio, halogen, cyano, nitro and C₁-C₈alkoxycarbonyl; or
R₁ is hydrogen, C₁-C₈alkyl or C₃-C₈cycloalkyl; or
R₁ is hydrogen or C₁-C₄alkyl; or
R₂ and R₃ are independently of each other hydrogen or C₁-C₄alkyl; or
R₂ and R₃ are hydrogen; or
R₄ is C₁-C₈alkyl, C₂-C₈alkenyl, or C₂-C₈alkynyl; or
R₄ is C₁-C₆alkyl; or
R₄ is C₁-C₄alkyl, or
R₄ is methyl or ethyl, especially methyl; or
R₅, R₆, R₇ and R₈ are independently of each other hydrogen or C₁-C₄alkyl; or
R₅, R₆ and R₇ are hydrogen and R₈ is hydrogen, methyl or ethyl, preferably methyl; or
R₅, R₆, R₇ and R₈ are hydrogen; or
R₉ is hydrogen, C₁-C₄alkyl, C₁-C₄haloalkyl, C₃-C₄alkenyl or C₃-C₄alkynyl; or
R₉ is hydrogen or C₁-C₄alkyl; or
R₉ is hydrogen; or
R₁₀ is aryl or heteroaryl, each optionally substituted with substituents selected from the group comprising alkyl, alkenyl, alkynyl, cycloalkyl, cycloalkyl-alkyl, phenyl and phenylalkyl, where all these groups may be substituted with one or more halogen atoms; alkoxy; alkenyloxy; alkynyloxy; alkoxy-alkyl; haloalkoxy; alkylthio; haloalkylthio; alkylsulfonyl; formyl; alkanoyl; hydroxy; cyano; nitro; amino; alkylamino; dialkylamino; carboxyl; alkoxycarbonyl; alkenyloxycarbonyl and alkynyloxycarbonyl; or
R₁₀ is phenyl, naphthyl or biphenyl, each optionally substituted by one to three substituents selected from the group comprising C₁-C₈alkyl, C₂-C₈alkenyl, C₂-C₈alkynyl, C₁-C₈haloalkyl, C₁-C₈alkoxy, C₁-C₈haloalkoxy, C₁-C₈alkylthio, C₁-C₈haloalkylthio, C₁-C₈alkylsulfonyl, halogen, cyano, nitro and C₁-C₈alkoxycarbonyl; or
R₁₀ is phenyl, naphthyl, 1,3-biphenyl or 1,4-biphenyl, each optionally substituted by one to three substituents selected from the group comprising C₁-C₈alkyl, C₁-C₈haloalkyl, C₁-C₈alkoxy, C₁-C₈haloalkoxy, C₁-C₈alkylthio, C₁-C₈haloalkylthio, halogen, cyano, nitro and C₁-C₈alkoxycarbonyl; or
Z is optionally substituted aryloxy wherein the aryl may be optionally substituted by one or more substituents selected from the group comprising halogen, C₁-C₈alkoxy, C₂-C₈alkenyloxy, C₂-C₈alkynyloxy, C₁-C₈alkoxy-C₁-C₈alkyl, C₁-C₈haloalkoxy, C₁-C₈alkylthio, C₁-C₈haloalkylthio, C₁-C₈alkylsulfonyl, formyl, C₂-C₈alkanoyl, hydroxy, halogen, cyano, nitro, amino, C₁-C₈alkylamino, di-C₁-C₈alkylamino, carboxyl and C₁-C₈alkoxycarbonyl; or is optionally substituted C₁-C₈alkoxy, optionally substituted C₂-C₈alkenyloxy or optionally substituted C₂-C₈alkynyloxy wherein each alkyl, alkenyl or alkynyl group may carry one or more substituents selected from the group comprising halogen, C₁-C₄alkyl, C₁-C₄alkoxy, C₁-C₄alkylthio, C₃-C₆cycloalkyl, nitro, cyano, hydroxy, phenyl, mercapto, C₁-C₄alkylcarbonyl and C₁-C₄alkoxycarbonyl; or
Z is C₁-C₈alkoxy, C₂-C₈alkenyloxy, C₂-C₈alkynyloxy, C₁-C₈alkoxy-C₁-C₈alkoxy, C₂-C₈alkenyloxy-C₁-C₈alkoxy, C₂-C₈alkynyloxy-C₁-C₈alkoxy, C₁-C₈haloalkoxy or C₃-C₈cycloalkyl-C₁-C₈alkoxy; or
Z is C₁-C₈alkoxy, C₂-C₈alkenyloxy, C₂-C₈alkynyloxy or C₁-C₄alkoxy-C₁-C₂alkoxy; or
Z is C₁-C₈alkoxy, C₂-C₆alkenyloxy or C₂-C₆alkynyloxy.

One preferred subgroup of the compounds of formula I consists of those compounds wherein R₉ is hydrogen, and Z is C₁-C₈alkoxy, C₂-C₆alkenyloxy or C₂-C₆alkynyloxy.

Further preferred subgroups are those wherein
R₁ is hydrogen, alkyl, cycloalkyl, phenyl or naphthyl; phenyl and naphthyl being optionally substituted by substituents selected from the group comprising alkyl, alkenyl, alkynyl, cycloalkyl, cycloalkyl-alkyl, phenyl and phenylalkyl, where all these groups may in turn be substituted by one or several halogens; alkoxy, alkenyloxy, alkynyloxy; alkoxy-alkyl; haloalkoxy; alkylthio; haloalkylthio; alkylsulfonyl; formyl; alkanoyl; hydroxy; halogen; cyano; nitro; amino; alkylamino; dialkylamino; carboxyl; alkoxycarbonyl; alkenyloxycarbonyl; or alkynyloxycarbonyl; and R₄ is alkyl; and R₁₀ is aryl or heteroaryl, each optionally substituted by substituents selected from to group comprising alkyl, alkenyl, alkynyl, cycloalkyl, cycloalkyl-alkyl, phenyl and phenylalkyl, where all these groups may be substituted by one or several halogen; alkoxy; alkenyloxy; alkynyloxy; alkoxy-alkyl; haloalkoxy; alkylthio; haloalkylthio; alkylsulfonyl; formyl; alkanoyl; hydroxy; cyano; nitro; amino; alkylamino; dialkylamino; carboxyl; alkoxycarbonyl; alkenyloxycarbonyl and alkynyloxycarbonyl; and Z is C₁-C₈alkoxy, C₂-C₆alkenyloxy or C₂-C₆alkynyloxy ; or
R₁ is hydrogen, C₁-C₈alkyl, C₃-C₈cycloalkyl, phenyl or naphthyl; phenyl and naphthyl being optionally substituted by one to three substituents selected from the group comprising C₁-C₈alkyl, C₂-C₈alkenyl, C₂-C₈alkynyl, C₁-C₈haloalkyl, C₁-C₈alkoxy, C₁-C₈haloalkoxy,
C₁-C₈alkylthio, C₁-C₈haloalkylthio, C₁-C₈alkylsulfonyl, halogen, cyano, nitro and C₁-C₈alkoxycarbonyl; and R₂, R₃, R₅, R₆, and R₇ are hydrogen; and R₄ and R₈ are independently C₁-C₆alkyl; and R₁₀ is phenyl, naphthyl, 1,3-biphenyl or 1,4-biphenyl, each optionally substituted by one to three substituents selected from the group comprising C₁-C₈alkyl, C₂-C₈alkenyl, C₂-C₈alkynyl, C₁-C₈haloalkyl, C₁-C₈alkoxy, C₁-C₈haloalkoxy, C₁-C₈alkylthio, C₁-C₈haloalkylthio, C₁-C₈alkylsulfonyl, halogen, cyano, nitro and C₁-C₈alkoxycarbonyl; and R₉ is hydrogen or C₁-C₄alkyl; and Z is C₁-C₈alkoxy, C₂-C₆alkenyloxy or C₂-C₆alkynyloxy; or
R₁ is hydrogen, C₁-C₈alkyl, phenyl optionally substituted by one to three substituents selected from the group comprising C₁-C₈alkyl, C₁-C₈haloalkyl, C₁-C₈alkoxy, C₁-C₈haloalkoxy, C₁-C₈alkylthio, C₁-C₈haloalkylthio, halogen, cyano, nitro and C₁-C₈alkoxycarbonyl; and R₂, R₃, R₅, R₆, and R₇ are hydrogen; and R₄ and R₈ are each independently methyl or ethyl; and R₁₀ is phenyl, naphthyl, 1,3-biphenyl or 1,4-biphenyl, each optionally substituted by one to three substituents selected from the group comprising C₁-C₈alkyl, C₁-C₈haloalkyl, C₁-C₈alkoxy, C₁-C₈haloalkoxy, C₁-C₈alkylthio, C₁-C₈haloalkylthio, halogen, cyano, nitro and C₁-C₈alkoxycarbonyl; R₉ is hydrogen and Z is C₁-C₈alkoxy, C₂-C₆alkenyloxy or C₂-C₆alkynyloxy.

Other preferred subgroups of the compounds of formula I are those wherein
R₁ is hydrogen, alkyl, cycloalkyl, phenyl or naphthyl; phenyl and naphthyl being optionally substituted by substituents selected from the group comprising alkyl, alkenyl, alkynyl, cycloalkyl, cycloalkyl-alkyl, phenyl and phenylalkyl, where all these groups may in turn be substituted by one or several halogens; alkoxy, alkenyloxy, alkynyloxy; alkoxy-alkyl; haloalkoxy; alkylthio; haloalkylthio; alkylsulfonyl; formyl; alkanoyl; hydroxy; halogen; cyano; nitro; amino; alkylamino; dialkylamino; carboxyl; alkoxycarbonyl; alkenyloxycarbonyl; or alkynyloxycarbonyl; and
R₂ and R₃ are independently of each other hydrogen or C₁-C₄alkyl; and
R₄ is C₁-C₈alkyl, C₂-C₈alkenyl, or C₂-C₈alkynyl; and
R₅, R₆, R₇ and R₈ are independently of each other hydrogen or C₁-C₄alkyl; and
R₉ is hydrogen, C₁-C₄alkyl, C₁-C₄haloalkyl, C₃-C₄alkenyl or C₃-C₄alkynyl; and
R₁₀ is aryl or heteroaryl, each optionally substituted with substituents selected from to group comprising alkyl, alkenyl, alkynyl, cycloalkyl, cycloalkyl-alkyl, phenyl and phenylalkyl, where all these groups may be substituted with one or more substituents selected from the group comprising halogen; alkoxy, alkenyloxy, alkynyloxy; alkoxy-alkyl; haloalkoxy; alkylthio; haloalkylthio; alkylsulfonyl; formyl; alkanoyl; hydroxy; cyano; nitro; amino; alkylamino; dialkylamino; carboxyl; alkoxycarbonyl; alkenyloxycarbonyl and alkynyloxycarbonyl; and
Z is optionally substituted aryloxy wherein in each the aryl may be optionally substituted by one or more substituents selected from the group comprising halogen, C₁-C₈alkoxy, C₂-C₈alkenyloxy, C₂-C₈alkynyloxy, C₁-C₈alkoxy-C₁-C₈alkyl, C₁-C₈haloalkoxy, C₁-C₈alkylthio, C₁-C₈haloalkylthio, C₁-C₈alkylsulfonyl, formyl, C₂-C₈alkanoyl, hydroxy, halogen, cyano, nitro, amino, C₁-C₈alkylamino, di-C₁-C₈alkylamino, carboxyl and C₁-C₈alkoxycarbonyl; or is optionally substituted C₁-C₈alkoxy, optionally substituted C₂-C₈alkenyloxy or optionally substituted C₂-C₈alkynyloxy, wherein each alkyl, alkenyl or alkynyl group may carry one or more substituents selected from the group comprising halogen, C₁-C₄alkyl, C₁-C₄alkoxy, C₁-C₄alkylthio, C₃-C₆cycloalkyl, nitro, cyano, hydroxy, phenyl, mercapto, C₁-C₄alkylcarbonyl and C₁-C₄alkoxycarbonyl; or wherein
R₁ is hydrogen, C₁-C₈alkyl, C₃-C₈cycloalkyl, phenyl or naphthyl; phenyl and naphthyl being optionally substituted by one to three substituents selected from the group comprising C₁-C₈alkyl, C₂-C₈alkenyl, C₂-C₈alkynyl, C₁-C₈haloalkyl, C₁-C₈alkoxy, C₁-C₈haloalkoxy, C₁-C₈alkylthio, C₁-C₈haloalkylthio, C₁-C₈alkylsulfonyl, halogen, cyano, nitro and C₁-C₈alkoxycarbonyl; and
R₂ and R₃ are hydrogen; and
R₄ is C₁-C₆alkyl; and
R₅, R₆ and R₇ are hydrogen and R₈ is hydrogen, methyl or ethyl, preferably methyl; and
R₉ is hydrogen or C₁-C₄alkyl; and
R₁₀ is phenyl, naphthyl or biphenyl, each optionally substituted by one to three substituents selected from the group comprising C₁-C₈alkyl, C₂-C₈alkenyl, C₂-C₈alkynyl, C₁-C₈haloalkyl, C₁-C₈alkoxy, C₁-C₈haloalkoxy, C₁-C₈alkylthio, C₁-C₈haloalkylthio, C₁-C₈alkylsulfonyl, halogen, cyano, nitro and C₁-C₈alkoxycarbonyl; and
Z is C₁-C₈alkoxy, C₂-C₈alkenyloxy, C₁-C₈alkynyloxy, C₁-C₈alkoxy-C₁-C₈alkoxy, C₂-C₈alkenyloxy-C₁-C₈alkoxy, C₂-C₈alkynyloxy-C₁-C₈alkoxy, C₁-C₈haloalkoxy or C₃-C₈cycloalkyl-C₁-C₈alkoxy; or wherein
R₁ is hydrogen, C₁-C₈alkyl or phenyl optionally substituted by one to three substituents selected from the group comprising C₁-C₈alkyl, C₁-C₈haloalkyl, C₁-C₈alkoxy, C₁-C₈haloalkoxy, C₁-C₈alkylthio, C₁-C₈haloalkylthio, halogen, cyano, nitro and C₁-C₈alkoxycarbonyl; and
R₂ and R₃ are hydrogen; and
R₄ is C₁-C₄alkyl, and
R₅, R₆ and R₇ are hydrogen and R₈ is hydrogen or methyl; and
R₉ is hydrogen; and
R₁₀ is phenyl, naphthyl, 1,3-biphenyl or 1,4-biphenyl, each optionally substituted by one to three substituents selected from the group comprising C₁-C₈alkyl, C₁-C₈haloalkyl, C₁-C₈alkoxy, C₁-C₈haloalkoxy, C₁-C₈alkylthio, C₁-C₈haloalkylthio, halogen, cyano, nitro and C₁-C₈alkoxycarbonyl; and
Z is C₁-C₈alkoxy, C₂-C₈alkenyloxy, C₂-C₈alkynyloxy or C₁-C₄alkoxy-C₁-C₂alkoxy; or wherein
R₁ is hydrogen, C₁-C₈alkyl or C₃-C₈cycloalkyl; and
R₂, R₃, R₅, R₆, R₇, R₈ and R₉ are hydrogen; and
R₄ is methyl or ethyl; and
R₁₀ is phenyl, naphthyl, 1,3-biphenyl or 1,4-biphenyl, each optionally substituted by one to three substituents selected from the group comprising C₁-C₈alkyl, C₁-C₈haloalkyl, C₁-C₈alkoxy, C₁-C₈haloalkoxy, C₁-C₈alkylthio, C₁-C₈haloalkylthio, halogen, cyano, nitro and C₁-C₈alkoxycarbonyl; and
Z is C₁-C₈alkoxy, C₂-C₆alkenyloxy or C₂-C₆alkynyloxy.

Preferred individual compounds are:
(S)-2-(4-bromo-phenyl)-2-methoxy-N-[2-(3-methoxy-4-prop-2-ynyloxy-phenyl)-ethyl]-acetamide,
(S)-2-(4-chloro-phenyl)-2-methoxy-N-[2-(3-methoxy-4-prop-2-ynyloxy-phenyl)-ethyl]-acetamide,
(S)-2-(3,4-dichloro-phenyl)-2-methoxy-N-[2-(3-methoxy-4-prop-2-ynyloxy-phenyl)-ethyl]-acetamide,
(S)-2-(4-bromo-phenyl)-2-ethoxy-N-[2-(3-methoxy-4-prop-2-ynyloxy-phenyl)-ethyl]-acetamide,
(S)-2-(4-chloro-phenyl)-2-ethoxy-N-[2-(3-methoxy-4-prop-2-ynyloxy-phenyl)-ethyl]-acetamide,
(S)-2-(3,4-dichloro-phenyl)-2-ethoxy-N-[2-(3-methoxy-4-prop-2-ynyloxy-phenyl)-ethyl]-acetamide,
(S)-2-(4-bromo-phenyl)-N-[2-(3-methoxy-4-prop-2-ynyloxy-phenyl)-ethyl]-2-propoxy-acetamide,
(S)-2-(4-chloro-phenyl)-N-[2-(3-methoxy-4-prop-2-ynyloxy-phenyl)-ethyl]-2-propoxy-acetamide,
(S)-2-(3,4-dichloro-phenyl)-N-[2-(3-methoxy-4-prop-2-ynyloxy-phenyl)-ethyl]-2-propoxy-acetamide,
(S)-2-(4-bromo-phenyl)-2-cyclopropylmethoxy-N-[2-(3-methoxy-4-prop-2-ynyloxy-phenyl)-ethyl]-acetamide,
(S)-2-(4-chloro-phenyl)-2-cyclopropylmethoxy-N-[2-(3-methoxy-4-prop-2-ynyloxy-phenyl)-ethyl]-acetamide,
(S)-2-cyclopropylmethoxy-2-(3,4-dichloro-phenyl)-N-[2-(3-methoxy-4-prop-2-ynyloxy-phenyl)-ethyl]-acetamide,
(S)-2-(4-bromo-phenyl)-2-ethoxymethoxy-N-[2-(3-methoxy-4-prop-2-ynyloxy-phenyl)-ethyl]-acetamide,
(S)-2-(4-chloro-phenyl)-2-ethoxymethoxy-N-[2-(3-methoxy-4-prop-2-ynyloxy-phenyl)-ethyl]-acetamide,
(S)-2-(3,4-dichloro-phenyl)-2-ethoxymethoxy-N-[2-(3-methoxy-4-prop-2-ynyloxy-phenyl)-ethyl]-acetamide,
(S)-2-allyloxy-2-(4-bromo-phenyl)-N-[2-(3-methoxy-4-prop-2-ynyloxy-phenyl)-ethyl]-acetamide,
(S)-2-allyloxy-2-(4-chloro-phenyl)-N-[2-(3-methoxy-4-prop-2-ynyloxy-phenyl)-ethyl]-acetamide,
(S)-2-allyloxy-2-(3,4-dichloro-phenyl)-N-[2-(3-methoxy-4-prop-2-ynyloxy-phenyl)-ethyl]-acetamide,
(S)-2-(4-bromo-phenyl)-2-(but-2-enyloxy)-N-[2-(3-methoxy-4-prop-2-ynyloxy-phenyl)-ethyl]-acetamide,
(S)-2-(but-2-enyloxy)-2-(4-chloro-phenyl)-N-[2-(3-methoxy-4-prop-2-ynyloxy-phenyl)-ethyl]-acetamide,
(S)-2-(but-2-enyloxy)-2-(3,4-dichloro-phenyl)-N-[2-(3-methoxy-4-prop-2-ynyloxy-phenyl)-ethyl]-acetamide,
(S)-2-(4-bromo-phenyl)-N-[2-(3-methoxy-4-prop-2-ynyloxy-phenyl)-ethyl]-2-prop-2-ynyloxy-acetamide,
(S)-2-(4-chloro-phenyl)-N-[2-(3-methoxy-4-prop-2-ynyloxy-phenyl)-ethyl]-2-prop-2-ynyloxy-acetamide,
(S)-2-(3,4-dichloro-phenyl)-N-[2-(3-methoxy-4-prop-2-ynyloxy-phenyl)-ethyl]-2-prop-2-ynyloxy-acetamide,
(S)-2-biphenyl-4-yl-N-[2-(3-methoxy-4-prop-2-ynyloxy-phenyl)-ethyl]-2-prop-2-ynyloxy-acetamide,
(S)-N-[2-(3-methoxy-4-prop-2-ynyloxy-phenyl)-ethyl]-2-naphthalen-2-yl-2-prop-2-ynyloxy-acetamide,
(S)-N-[2-(3-methoxy-4-prop-2-ynyloxy-phenyl)-ethyl]-2-prop-2-ynyloxy-2-*p*-tolyl-acetamide, 2-(4-ethyl-phenyl)-N-[2-(3-methoxy-4-prop-2-ynyloxy-phenyl)-ethyl]-2-prop-2-ynyloxy-acetamide,
(S)-N-[2-(3-methoxy-4-prop-2-ynyloxy-phenyl)-ethyl]-2-prop-2-ynyloxy-2-(4-trifluoromethyl-phenyl)-acetamide,
(S)-2-(4-bromo-phenyl)-2-but-2-ynyloxy-N-[2-(3-methoxy-4-prop-2-ynyloxy-phenyl)-ethyl]-acetamide,
(S)-2-but-2-ynyloxy-2-(4-chloro-phenyl)-N-[2-(3-methoxy-4-prop-2-ynyloxy-phenyl)-ethyl]-acetamide,
(S)-2-but-2-ynyloxy-2-(3,4-dichloro-phenyl)-N-[2-(3-methoxy-4-prop-2-ynyloxy-phenyl)-ethyl]-acetamide,
(S)-2-(4-bromo-phenyl)-N-[2-(3-methoxy-4-pent-2-ynyloxy-phenyl)-ethyl]-2-prop-2-ynyloxy-acetamide,
(S)-2-(4-chloro-phenyl)-N-[2-(3-methoxy-4-pent-2-ynyloxy-phenyl)-ethyl]-2-prop-2-ynyloxy-acetamide,
(S)-2-(3,4-dichloro-phenyl)-N-[2-(3-methoxy-4-pent-2-ynyloxy-phenyl)-ethyl]-2-prop-2-ynyloxy-acetamide,
(S)-2-(4-fluoro-phenyl)-N-[2-(3-methoxy-4-prop-2-ynyloxy-phenyl)-ethyl]-2-prop-2-ynyloxy-acetamide,
(S)-2-(3,4-difluoro-phenyl)-N-[2-(3-methoxy-4-prop-2-ynyloxy-phenyl)-ethyl]-2-prop-2-ynyloxy-acetamide,
(S)-2-(4-chloro-3-fluoro-phenyl)-N-[2-(3-methoxy-4-prop-2-ynyloxy-phenyl)-ethyl]-2-prop-2-ynyloxy-acetamide, and
(S)-2-(3-chloro-4-fluoro-phenyl)-N-[2-(3-methoxy-4-prop-2-ynyloxy-phenyl)-ethyl]-2-prop-2-ynyloxy-acetamide.

Certain racemic mandelic acid derivatives have been proposed for controlling plant-destructive fungi (for example in WO 94/29267, in WO 96/17840, in WO 00/41998 and in WO 01/87822 (PCT/EP01/05530)). The action of those racemic derivatives is not, however, satisfactory in all aspects of agricultural needs. Surprisingly, with the enantiomeric form of the compounds of formula I, new microbiocides having a high level of activity have been found.

The enantiomeric propargylether derivatives of formula I and displayed subformulae and intermediates may be obtained either by separation methods from the racemic products having the same molecular design, or by stereoselective synthesis methods starting from optically active pure isomers of the starting materials via defined stereoselective processes and pure optical isomers of the intermediates according to one of the processes of Schemes 1 and 2. 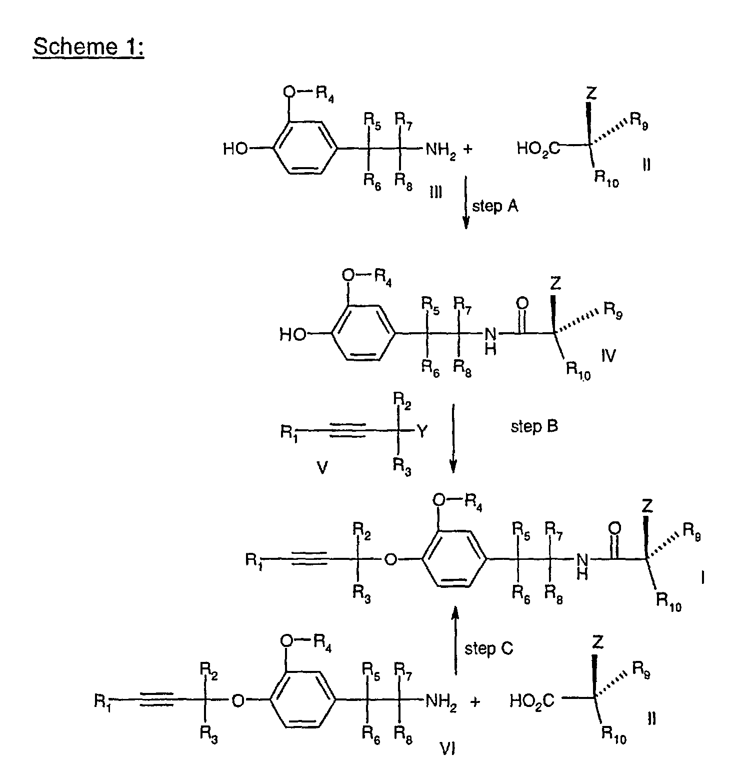
Step A: The acid of formula II or a carboxy-activated derivative of an acid of formula II wherein R₉, R₁₀ and Z are as defined for formula I is reacted with an amine of formula III wherein R₄, R₅, R₆, R₇ and R₈ are as defined for formula I, optionally in the presence of a base and optionally in the presence of a diluting agent.
   Carboxy-activated derivatives of the acid of formula II are all compounds having an activated carboxyl group like an acid halide, such as an acid chloride, like symmetrical or mixed anhydrides, such as mixed anhydrides with O-alkylcarbonates, like activated esters, such as p-nitrophenylesters or N-hydroxysuccinimidesters, as well as in-situ-formed activated forms of the acid of formula II with condensating agents, such as dicyclohexylcarbodiimide, carbonyldiimidazole, benzotriazol-1-yloxy-tris(dimethylamino)phosphonium hexafluorophosphate, O-benzotriazol-1-yl-N,N,N',N'-bis(pentamethylene)uronium hexafluorophosphate, O-benzotriazol-1-yl-N,N,N',N'-bis(tetramethylene)uronium hexafluorophosphate, O-benzotriazol-1-yl-N,N,N',N'-tetramethyluronium hexafluorophosphate or benzotriazol-1-yloxy-tripyrrolidinophosphonium hexafluorophosphate. The mixed anhydrides of the acids of the formula II may be prepared by reaction of an acid of formula II with chloroformic acid esters like chloroformic acid alkylesters, such as ethyl chloroformate or isobutyl chloroformate, optionally in the presence of an organic or inorganic base like a tertiary amine, such as triethylamine, N,N-diisopropyl-ethylamine, pyridine, N-methyl-piperidine or N-methyl-morpholine.
   The present reaction is preferably performed in a solvent like aromatic, non-aromatic or halogenated hydrocarbons, such as chlorohydrocarbons e.g. dichloromethane or toluene; ketones e.g. acetone; esters e.g. ethyl acetate; amides e.g. N,N-dimethylformamide; nitriles e.g. acetonitrile; or ethers e.g. diethylether, tert-butyl-methylether, dioxane or tetrahydrofurane or water. It is also possible to use mixtures of these solvents. The reaction is performed optionally in the presence of an organic or inorganic base like a tertiary amine, e.g. triethylamine, N,N-diisopropyl-ethylamine, pyridine, N-methyl-piperidine or N-methyl-morpholine, like a metal hydroxide or a metal carbonate, preferentially an alkali hydroxide or an alkali carbonate, such as lithium hydroxide, sodium hydroxide or potassium hydroxide at temperatures ranging from -80°C to +150°C, preferentially at temperatures ranging from -40°C to +40°C.
Step B: The compounds of formula I may then finally be prepared by reacting a phenol of formula IV wherein R₄, R₅, R₆, R₇, R₈, R₉, R₁₀ and Z are as defined for formula I with a compound of formula V wherein R₁, R₂ and R₃ are as defined for formula I and wherein Y is a leaving group like a halide such as a chloride or bromide or a sulfonic ester such as a tosylate, mesylate or triflate.
   The reaction is advantageously performed in a solvent like aromatic, non-aromatic or halogenated hydrocarbons, such as chlorohydrocarbons e.g. dichloromethane or toluene; ketones e.g. acetone or 2-butanone; esters e.g. ethyl acetate; ethers e.g. diethylether, tert-butyl-methylether, dioxane or tetrahydrofurane, amides e.g. dimethylformamide, nitriles e.g. acetonitrile, alcohols e.g. methanol, ethanol, isopropanol, n-butanol or tert-butanol, sulfoxides e.g. dimethylsulfoxide or water. It is also possible to use mixtures of these solvents. The reaction is performed optionally in the presence of an organic or inorganic base like a tertiary amine, such as triethylamine, N,N-diisopropyl-ethylamine, pyridine, N-methyl-piperldine or N-methyl-morpholine, like a metal hydroxide, a metal carbonate or a metal alkoxide, preferentially an alkali hydroxide, an alkali carbonate or an alkali alkoxide, such as lithium hydroxide, sodium hydroxide, potassium hydroxide, sodium carbonate, potassium carbonate, sodium methoxide, potassium methoxide, sodium ethoxide, potassium ethoxide, sodium tert-butoxide or potassium tert-butoxide at temperatures ranging from -80°C to +200°C, preferentially at temperatures ranging from 0°C to +120°C.
Step C: Alternatively to step A and step B, the (S)-form of an acid of formula II or a carboxy-activated derivative of an acid of formula II wherein R₉, R₁₀ and Z are as defined for formula I is reacted with an amine of formula VI wherein R₁, R₂, R₃, R₄, R₅, R₆, R₇ and R₈ are as defined for formula I under the same conditions as defined for step A, optionally in the presence of a base and optionally in the presence of a diluting agent. 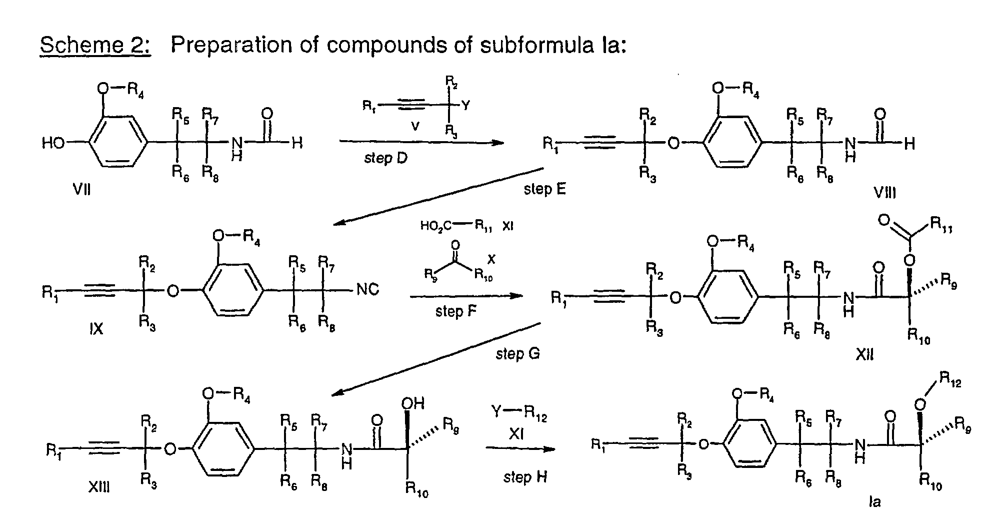
Step D: A compound of formula VII wherein R₄, R₅, R₆, R₇ and R₈ are as defined for formula I is alkylated with a compound of formula V (see Scheme 1) wherein R₁, R₂, R₃ and Y are as defined for Scheme 1 under the same conditions as defined for step B in Scheme 1.
Step E: A compound of formula VIII wherein R₁, R₂, R₃, R₄, R₅, R₆, R₇ and R₈ are as defined for formula I is dehydrated to an isocyanide of formula IX wherein R₁, R₂, R₃, R₄, R₅, R₆, R₇ and R₈ are as defined for formula I under conditions known per se (D. Seebach, G. Adam, T. Gees, M. Schiess, W. Weigang, *Chem. Ber.* **1988,** *121*, 507).
Step F: An isocyanide of formula IX wherein R₁, R₂, R₃, R₄, R₅, R₆, R₇ and R₈ are as defined for formula I is reacted in a three-component Passerini reaction (J. March, *Advanced Organic Chemistry*, 4th ed., Wiley, **1992,** p. 980) with an aldehyde or ketone of formula X, wherein R₉ and R₁₀ are as defined for formula I in the presence of a chiral carboxylic acid XI, typically an amino acid or a sugar acid like a glyconic acid or a glycuronic acid, preferably a glycuronic acid like 1,2,3,4-tetra-O-acetyl-α-D-galacturonic acid or 1,2,3,4-tetra-O-acetyl-α-D-glucuronic acid to give a O-acyl-α-hydroxy amide of formula XII, wherein R₁, R₂, R₃, R₄, R₅, R₆, R₇, R₈, R₉ and R₁₀ are as defined for formula I. The reaction is advantageously performed in a solvent like aromatic, non-aromatic or halogenated hydrocarbons, such as chlorohydrocarbons e.g. dichloromethane or toluene; ketones e.g. acetone or 2-butanone; esters e.g. ethyl acetate; ethers e.g. diethylether, tert-butyl-methylether, dioxane or tetrahydrofuran, amides e.g. dimethylformamide, nitriles e.g. acetonitrile, alcohols e.g. methanol, ethanol, isopropanol, n-butanol or tert-butanol, sulfoxides e.g. dimethylsulfoxide or water. It is also possible to use mixtures of these solvents.
Step G: The O-acyl-α-hydroxy amide of formula XII wherein R₁, R₂, R₃, R₄, R₅, R₆, R₇, R₈, R₉, R₁₀ and R₁₁ are as defined above is hydrolyzed to a an α-hydroxy amide of formula XIII, wherein R₁, R₂, R₃, R₄, R₅, R₆, R₇ , R₈, R₉ and R₁₀ are as defined for formula I under classical conditions (J. March, *Advanced Organic Chemistry*, 4th ed., Wiley, **1992**).
Step H: The α-hydroxy amide of formula XIII wherein R₁, R₂, R₃, R₄, R₅, R₆, R₇, R₈, R₉ and R₁₀ are as defined for formula I is reacted with a compound XIV wherein R₁₂ is alkyl, alkenyl or alkynyl and Y is a leaving group like a halide such as a chloride or bromide or a sulfonic ester such as a tosylate, mesylate or triflate to a compound of formula la wherein R₁, R₂, R₃, R₄, R₅, R₆, R₇, R₈, R₉ and R₁₀ are as defined for formula I and R₁₂ is alkyl, alkenyl or alkynyl under the same conditions as defined for step B in Scheme 1.

The compounds of formula I are oils or solids at room temperature and are distinguished by valuable microbiocidal properties. They can be used in the agricultural sector or related fields preventatively and curatively in the control of plant-destructive microorganisms. The compounds of formula I according to the invention are distinguished at low rates of concentration not only by outstanding microbiocidal, especially fungicidal, activity but also by being especially well tolerated by plants.

Surprisingly, it has now been found that the compounds of formula I have for practical purposes a very advantageous microbiocidal spectrum in the control of phytopathogenic microorganisms, especially fungi. They possess very advantageous curative and preventive properties and are used in the protection of numerous crop plants. With the compounds of formula I it is possible to inhibit or destroy phytopathogenic microorganisms that occur on various crops of useful plants or on parts of such plants (fruit, blossom, leaves, stems, tubers, roots), while parts of the plants which grow later also remain protected, for example, against phytopathogenic fungi.

The novel compounds of formula I prove to be effective against specific genera of the fungus class Fungi imperfecti (e.g. Cercospora), Basidiomycetes (e.g. Puccinia) and Ascomycetes (e.g. Erysiphe and Venturia) and especially against Oomycetes (e.g. Plasmopara, Peronospora, Pythium and Phytophthora). They therefore represent in plant protection a valuable addition to the compositions for controlling phytopathogenic fungi. The compounds of formula I can also be used as dressings for protecting seed (fruit, tubers, grains) and plant cuttings from fungal infections and against phytopathogenic fungi that occur in the soil.

The invention relates also to compositions comprising compounds of formula I as active ingredient, especially plant-protecting compositions, and to the use thereof in the agricultural sector or related fields.

In addition, the present invention includes the preparation of those compositions, wherein the active ingredient is homogeneously mixed with one or more of the substances or groups of substances described herein. Also included is a method of treating plants which is distinguished by the application of the novel compounds of formula I or of the novel compositions.

Target crops to be protected within the scope of this invention comprise, for example, the following species of plants: cereals (wheat, barley, rye, oats, rice, maize, sorghum and related species); beet (sugar beet and fodder beet); pomes, stone fruit and soft fruit (apples, pears, plums, peaches, almonds, cherries, strawberries, raspberries and blackberries); leguminous plants (beans, lentils, peas, soybeans); oil plants (rape, mustard, poppy, olives, sunflowers, coconut, castor oil plants, cocoa beans, groundnuts); cucurbitaceae (marrows, cucumbers, melons); fibre plants (cotton, flax, hemp, jute); citrus fruit (oranges, lemons, grapefruit, mandarins); vegetables (spinach, lettuce, asparagus, cabbages, carrots, onions, tomatoes, potatoes, paprika); lauraceae (avocado, cinnamon, camphor) and plants such as tobacco, nuts, coffee, sugar cane, tea, pepper, vines, hops, bananas and natural rubber plants, and also ornamentals.

The compounds of formula I are normally used in the form of compositions and can be applied to the area or plant to be treated simultaneously or in succession with other active ingredients. Those other active ingredients may be fertilisers, micronutrient donors or other preparations that influence plant growth. It is also possible to use selective herbicides or insecticides, fungicides, bactericides, nematicides, molluscicides or mixtures of several of those preparations, if desired together with further carriers, surfactants or other application-promoting adjuvants customarily employed in formulation technology.

The compounds of formula I can be mixed with other fungicides, resulting in some cases in unexpected synergistic activities. Such mixtures are not limited to two active ingredients (one of formula I and one of the list of other fungicides), but to the contrary many comprise more than one active ingredient of the component of formula I and more than one other fungicide. Mixing components which are particularly suited for this purpose include e.g. azoles, such as azaconazole, BAY 14120, bitertanol, bromuconazole, cyproconazole, difenoconazole, diniconazole, epoxiconazole, fenbuconazole, fluquinconazole, flusilazole, flutriafol, hexaconazole, imazalil, imibenconazole, ipconazole, metconazole, myclobutanil, pefurazoate, penconazole, pyrifenox, prochloraz, propiconazole, simeconazole, tebuconazole, tetraconazole, triadimefon, triadimenol, triflumizole, triticonazole; pyrimidinyl carbinoles, such as ancymidol, fenarimol, nuarimol; 2-amino-pyrimidines, such as bupirimate, dimethirimol, ethirimol; morpholines, such as dodemorph, fenpropidine, fenpropimorph, spiroxamine, tridemorph; anilinopyrimidines, such as cyprodinil, mepanipyrim, pyrimethanil; pyrroles, such as fenpiclonil, fludioxonil; phenylamides, such as benalaxyl, furalaxyl, metalaxyl, R-metalaxyl, ofurace, oxadixyl; benzimidazoles, such as benomyl, carbendazim, debacarb, fuberidazole, thiabendazole; dicarboximides, such as chlozolinate, dichlozoline, iprodione, myclozoline, procymidone, vinclozoline; carboxamides, such as carboxin, fenfuram, flutolanil, mepronil, oxycarboxin, thifluzamide; guanidines, such as guazatine, dodine, iminoctadine; strobilurines, such as azoxystrobin, kresoxim-methyl, metominostrobin, SSF-129, trifloxystrobin, picoxystrobin, BAS 500F (proposed name pyraclostrobin), BAS 520; dithiocarbamates, such as ferbam, mancozeb, maneb, metiram, propineb, thiram, zineb, ziram; N-halomethylthiotetrahydrophthalimides, such as captafol, captan, dichlofluanid, fluoromides, folpet, tolyfluanid; Cu-compounds, such as Bordeaux mixture, copper hydroxide, copper oxychloride, copper sulfate, cuprous oxide, mancopper, oxine-copper; nitrophenol-derivatives, such as dinocap, nitrothal-isopropyl; organo-p-derivatives, such as edifenphos, iprobenphos, isoprothiolane, phosdiphen, pyrazophos, tolclofos-methyl; various others, such as acibenzolar-S-methyl, anilazine, benthiavalicarb, blasticidin-S, chinomethionate, chloroneb, chlorothalonil, cyflufenamid, cymoxanil, dichlone, diclomezine, dicloran, diethofencarb, dimethomorph, SYP-L190 (proposed name: flumorph), dithianon, ethaboxam, etridiazole, famoxadone, fenamidone, fenoxanil, fentin, ferimzone, fluazinam, flusulfamide, fenhexamid, fosetyl-aluminium, hymexazol, iprovalicarb, IKF-916 (cyazofamid), kasugamycin, methasulfocarb, metrafenone, nicobifen (new proposal: boscalid), pencycuron, phthalide, polyoxins, probenazole, propamocarb, pyroquilon, quinoxyfen, quintozene, sulfur, triazoxide, tricyclazole, triforine, validamycin, zoxamide (RH7281).

Some particularly interesting mixtures in view of technical value in the agricultural practice (comprising at least the one mentioned compound of formula I together with the above mentioned mentioned other fungicide, but not being limited thereto, i.e. such mixtures may comprise additional components according to needs when controlling certain fungi on certain crop plants) , having enhanced synergistic levels of fungicidal activity, or being especially well suited for the control of persistent or very damaging phytopathogenic fungi are among the following:
1) (S)-2-phenyl-N-[2-(3-methoxy-4-prop-2-ynyloxy-phenyl)-ethyl]-2-prop-2-ynyloxy-acetamide(compound E1.002), combined with any one active ingredient selected from cymoxanil, trifloxystrobin, azoxystrobin, picoxystrobin, chlorothalonil, metalaxyl, metalaxyl-M, pyraclostrobin (BAS500F), dimethomorph, fosetyl-Al, copper-salts, acibenzolar-S-methyl, fludioxonil, mancozeb, folpet, fluazinam, iprovalicarb, zoxamid and (S)-2-(methylsulfonyl-amino)-3-methyl-butyric acid N-[2-{3-methoxy-4-(3-(4-chlorophenyl)-2-propyn-1-yloxy)-phenyl}-ethyl]-amide; and
2) (S)-2-(4-chloro-phenyl)-N-[2-(3-methoxy-4-prop-2-ynyloxy-phenyl)-ethyl]-2-prop-2-ynyl-oxy-acetamide(compound E1.011), combined with any one active ingredient selected from cymoxanil, trifloxystrobin, azoxystrobin, picoxystrobin, chlorothalonil, metalaxyl, metalaxyl-M, pyraclostrobin (BAS500F), dimethomorph, fosetyl-Al, copper-salts, acibenzolar-S-methyl, fludioxonil, mancozeb, folpet, fluazinam, iprovalicarb, zoxamid and (S)-2-(methylsulfonyl-amino)-3-methyl-butyric acid N-[2-{3-methoxy-4-(3-(4-chlorophenyl)-2-propyn-1-yloxy)-phenyl}-ethyl]-amide, and
3) (S)-2-(4-bromo-phenyl)-N-[2-(3-methoxy-4-prop-2-ynyloxy-phenyl)-ethyl]-2-prop-2-yn-yloxy-acetamide (compound E1.022), combined with any one active ingredient selected from cymoxanil, trifloxystrobin, azoxystrobin, picoxystrobin, chlorothalonil, metalaxyl, metalaxyl-M, pyraclostrobin (BAS500F), dimethomorph, fosetyl-Al, copper-salts, acibenzolar-S-methyl, fludioxonil, mancozeb, folpet, fluazinam, iprovalicarb, zoxamid and (S)-2-(methylsulfonyl-amino)-3-methyl-butyric acid N-[2-{3-methoxy-4-(3-(4-chlorophenyl)-2-propyn-1-yloxy)-phenyl}-ethyl]-amide, and
4) (S)-2-(4-fluoro-phenyl)-N-[2-(3-methoxy-4-prop-2-ynyloxy-phenyl)-ethyl]-2-prop-2-ynyl-oxy-acetamide (compound E1.033), combined with any one active ingredient selected from cymoxanil, trifloxystrobin, azoxystrobin, picoxystrobin, chlorothalonil, metalaxyl, metalaxyl-M, pyraclostrobin (BAS500F), dimethomorph, fosetyl-Al, copper-salts, acibenzolar-S-methyl, fludioxonil, mancozeb, folpet, fluazinam, iprovalicarb, zoxamid and (S)-2-(methylsulfonyl-amino)-3-methyl-butyric acid N-[2-{3-methoxy-4-(3-(4-chlorophenyl)-2-propyn-1-yloxy)-phenyl}-ethyl]-amide, and
5) (S)-2-(4-tolyl)-N-[2-(3-methoxy-4-prop-2-ynyloxy-phenyl)-ethyl]-2-prop-2-ynyloxy-acetamide (compound E1.045), combined with any one active ingredient selected from cymoxanil, trifloxystrobin, azoxystrobin, picoxystrobin, chlorothalonil, metalaxyl, metalaxyl-M, pyraclostrobin (BAS500F), dimethomorph, fosetyl-Al, copper-salts, acibenzolar-S-methyl, fludioxonil, mancozeb, folpet, fluazinam, iprovalicarb, zoxamid and (S)-2-(methylsulfonyl-amino)-3-methyl-butyric acid N-[2-{3-methoxy-4-(3-(4-chlorophenyl)-2-propyn-1-yloxy)-phenyl}-ethyl]-amide, and
6) (S)-2-(4-ethyl-phenyl)-N-[2-(3-methoxy-4-prop-2-ynyloxy-phenyl)-ethyl]-2-prop-2-ynyl-oxy-acetamide (compound E1.053), combined with any one active ingredient selected from cymoxanil, trifloxystrobin, azoxystrobin, picoxystrobin, chlorothalonil, metalaxyl, metalaxyl-M, pyraclostrobin (BAS500F), dimethomorph, fosetyl-Al, copper-salts, acibenzolar-S-methyl, fludioxonil, mancozeb, folpet, fluazinam, iprovalicarb, zoxamid and (S)-2-(methylsulfonyl-amino)-3-methyl-butyric acid N-[2-{3-methoxy-4-(3-(4-chlorophenyl)-2-propyn-1-yloxy)-phenyl}-ethyl]-amide, and
7) (S)-2-(3,4-difluoro-phenyl)-N-[2-(3-methoxy-4-prop-2-ynyloxy-phenyl)-ethyl]-2-prop-2-ynyloxy-acetamide (compound E1.085), combined with any one active ingredient selected from cymoxanil, trifloxystrobin, azoxystrobin, picoxystrobin, chlorothalonil, metalaxyl, metalaxyl-M, pyraclostrobin (BAS500F), dimethomorph, fosetyl-Al, copper-salts, acibenzolar-S-methyl, fludioxonil, mancozeb, folpet, fluazinam, iprovalicarb, zoxamid and (S)-2-(methylsulfonyl-amino)-3-methyl-butyric acid N-[2-{3-methoxy-4-(3-(4-chlorophenyl)-2-propyn-1-yloxy)-phenyl}-ethyl]-amide, and
8) (S)-2-(3-fluoro-4-chloro-phenyl)-N-[2-(3-methoxy-4-prop-2-ynyloxy-phenyl)-ethyl]-2-prop-2-ynyloxy-acetamide (compound E1.091), combined with any one active ingredient selected from cymoxanil, trifloxystrobin, azoxystrobin, picoxystrobin, chlorothalonil, metalaxyl, metalaxyl-M, pyraclostrobin (BAS500F), dimethomorph, fosetyl-Al, copper-salts, acibenzolar-S-methyl, fludioxonil, mancozeb, folpet, fluazinam, iprovalicarb, zoxamid and (S)-2-(methylsulfonyl-amino)-3-methyl-butyric acid N-[2-{3-methoxy-4-(3-(4-chlorophenyl)-2-propyn-1-yloxy)-phenyl}-ethyl]-amide, and
9) (S)-2-(3,4-dichloro-phenyl)-N-[2-(3-methoxy-4-prop-2-ynyloxy-phenyl)-ethyl]-2-prop-2-ynyloxy-acetamide (compound E1.102), combined with any one active ingredient selected from cymoxanil, trifloxystrobin, azoxystrobin, picoxystrobin, chlorothalonil, metalaxyl, metalaxyl-M, pyraclostrobin (BAS500F), dimethomorph, fosetyl-Al, copper-salts, acibenzolar-S-methyl, fludioxonil, mancozeb, folpet, fluazinam, iprovalicarb, zoxamid and (S)-2-(methylsulfonyl-amino)-3-methyl-butyric acid N-[2-{3-methoxy-4-(3-(4-chlorophenyl)-2-propyn-1-yloxy)-phenyl}-ethyl]-amide.

In the above mentioned mixtures, the mixture ratio of the active ingredients is so selected that it reaches optional control of the phytopathogenic microorganism on the host plants. This ratio is in general between 100:1 and 1:100, preferably between 50: 1 and 1: 100, more preferably between 10:1 and 1:10, most preferably between 5:1 and 1: 10 of a compound of formula I vis-à-vis the second fungicide. The mixtures may not only comprise one of the listed combinational active ingredients, but may comprise more than one additional active ingredients selected from that specified group, thus forming for example 3-way- or even 4-way-mixtures.

Suitable carriers and surfactants may be solid or liquid and correspond to the substances ordinarily employed in formulation technology, such as e.g. natural or regenerated mineral substances, solvents, dispersants, wetting agents, tackifiers, thickeners, binders or fertilisers. Such carriers and additives are described, for example, in WO 95/30651.

A preferred method of applying a compound of formula I, or an agrochemical composition comprising at least one of those compounds, is application to the foliage (foliar application), the frequency and the rate of application depending upon the risk of infestation by the pathogen in question. The compounds of formula I may also be applied to seed grains (coating) either by impregnating the grains with a liquid formulation of the active ingredient or by coating them with a solid formulation.

The compounds of formula I are used in unmodified form or, preferably, together with the adjuvants conventionally employed in formulation technology, and are for that purpose advantageously formulated in known manner e.g. into emulsifiable concentrates, coatable pastes, directly sprayable or dilutable solutions, dilute emulsions, wettable powders, soluble powders, dusts, granules, and by encapsulation in e.g. polymer substances. As with the nature of the compositions, the methods of application, such as spraying, atomising, dusting, scattering, coating or pouring, are chosen in accordance with the intended objectives and the prevailing circumstances.

Advantageous rates of application are normally from 1 g to 2 kg of active ingredient (a.i.) per hectare (ha), preferably from 5 g to 1 kg a.i./ha, preferably from 25 g to 750 g a.i./ha, especially from 10 g to 500g a.i./ha, more preferably from 5 g to 500g a.i./ha. Most preferred is 5g to 250 g a.i./ha . When used as seed dressings, rates of from 0.001 g to 1.0 g of active ingredient per kg of seed are advantageously used.

The formulations, i.e. the compositions, preparations or mixtures comprising the compound(s) (active ingredient(s)) of formula I and, where appropriate, a solid or liquid adjuvant, are prepared in known manner, e.g. by homogeneously mixing and/or grinding the active ingredient with extenders, e.g. solvents, solid carriers and, where appropriate, surface-active compounds (surfactants).

Further surfactants customarily used in formulation technology will be known to the person skilled in the art or can be found in the relevant technical literature.

The agrochemical compositions usually comprise 0.01 to 99 % by weight, preferably 0.1 to 95 % by weight, of a compound of formula I, 99.99 to 1 % by weight, preferably 99.9 to 5 % by weight, of a solid or liquid adjuvant, and 0 to 25 % by weight, preferably 0.1 to 25 % by weight, of a surfactant.

Whereas commercial products will preferably be formulated as concentrates, the end user will normally employ dilute formulations.

The compositions may also comprise further ingredients, such as stabilisers, antifoams, viscosity regulators, binders and tackifiers, as well as fertilisers or other active ingredients for obtaining special effects.

The Examples which follow illustrate the invention described above, without limiting the scope thereof in any way. Temperatures are given in degrees Celsius. Ph stands for phenyl.

### Preparation Examples :

### Example E1: (S)-N-[2-(3-Methoxy-4-prop-2-ynyloxy-phenyl)-ethyl]-2-phenyl-2-prop-2-ynyloxy-acetamide

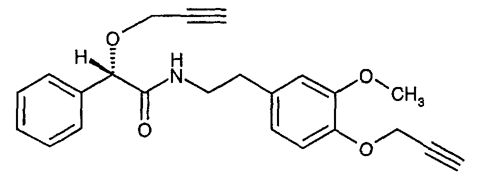

a) (S)-2-Hydroxy-*N*-[2-(3-methoxy-4-prop-2-ynyloxy-phenyl)-ethyl]-2-phenyl-acetamide 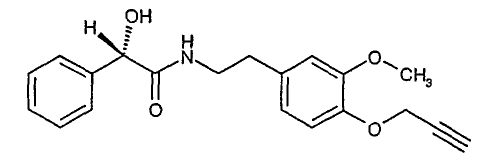 2-(3-Methoxy-4-prop-2-ynyloxy-phenyl)-ethylamine hydrochloride (8.5 g, 35 mol) and N,N-diisopropylethylamine (17 g, 0.13 mol) are dissolved in 100 ml of N,N-dimethylformamide. To this solution L-(+)-mandelic acid [(S)-α-hydroxyphenylacetic acid] (5.0 g, 33 mmol) and (benzotriazol-1-yloxy)-tris-(dimethylamino)-phosphonium hexafluorophosphate (16 g, 36 mmol) are added successively. The reaction mixture is stirred for 3 h at room temperature subsequently poured on ice-water and extracted several times with ethyl acetate. The combined organic layer is washed with brine, dried over sodium sulfate and evaporated in vacuo. The remaining oil is purified by chromatography on silicagel (ethyl acetate / hexane 1 : 1) to yield (S)-2-hydroxy-*N*-[2-(3-methoxy-4-prop-2-ynyloxy-phenyl)-ethyl]-2-phenylacetamide
   ¹H-NMR (300 MHz, CDCl₃): 2.72 (t, 1 H, C≡CH), 2.93 (q, 2H, CH₂CH₂), 3.72 (dt, 2H, CH₂CH₂), 4.01 (s, 3H, OCH₃), 4.97 (d, 2H, OCH₂), 5.20 (s, 1H, CHOH), 6.28 (bs, 1H, NH), 6.73 - 7.58 (m, 8H, CH arom.).
b) A 80 % solution of propargyl bromide in toluene (4.5 g, 30 mmol) is added slowly at room temperature to a mixture of (S)-2-hydroxy-*N*-[2-(3-methoxy-4-prop-2-ynyloxy-phenyl)-ethyl]-2-phenyl-acetamide (8.0 g, 24 mmol), 30 % sodium hydroxide solution (12 ml, 118 mmol) and catalytic amounts of tetrabutylammonium bromide (75 mg) in 40 ml of dichloromethane. The reaction is stirred for 16 hours at +40°C. Subsequently the mixture is evaporated and the residue is diluted with water and dichloromethane. The phases are separated and the aqueous phase is extracted three times with dichloromethane. The combined organic layer is washed with brine, dried over sodium sulfate and evaporated. The remaining oil is purified by chromatography on silica gel (ethyl acetate / hexane 1 : 1) to give (S)-*N*-[2-(3-methoxy-4-prop-2-ynyloxy-phenyl)-ethyl]-2-phenyl-2-prop-2-ynyloxy-acetamide.
   ¹H-NMR (300 MHz, CDCl₃): 2.41 (t, 1H, C≡CH), 2.46 (t, 1H, C≡CH), 2.73 (t, 2H, CH₂CH₂), 3.48 (m, 2H, CH₂CH₂), 3.79 (s, 3H, OCH₃), 3.91 (dd, 1H, OCH₂), 4.12 (dd, 1H, OCH₂), 4.71 (d, 2H, OCH₂), 4.93 (s, 1H, CHO), 6.62 - 7.30 (m, 9H, CH arom., NH). M.p.: 81 - 83 °C.

According to the procedures of Example E1 the compounds listed in table E1 are obtained.

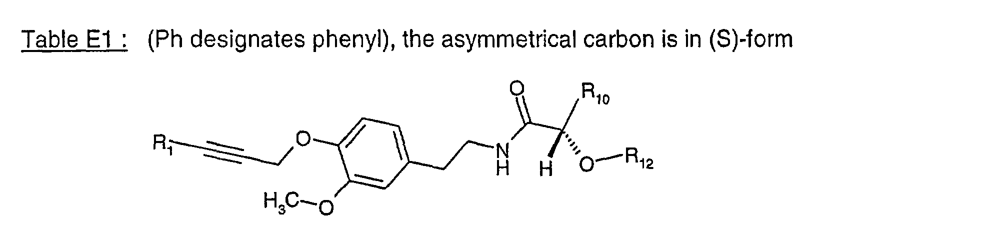

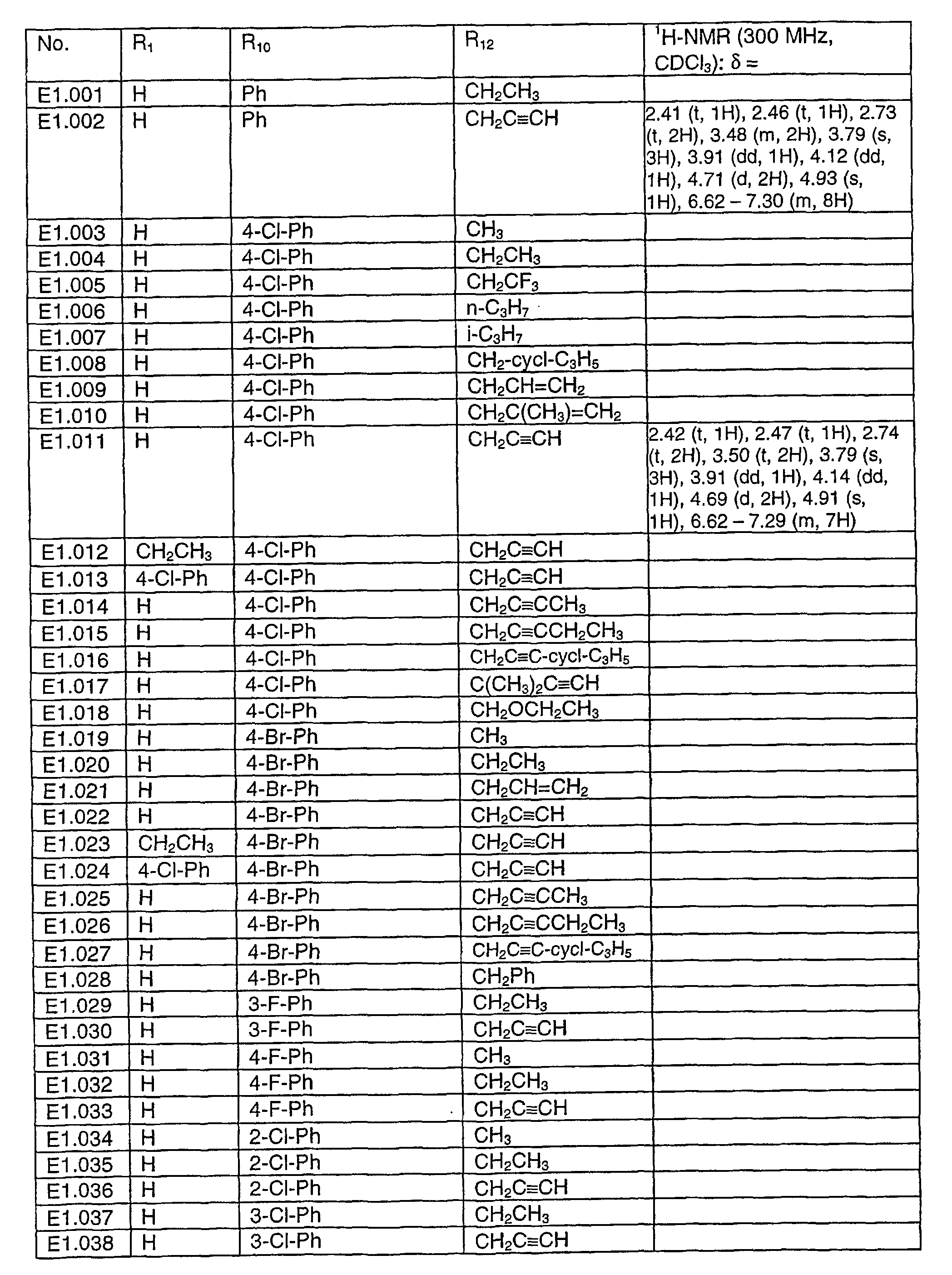

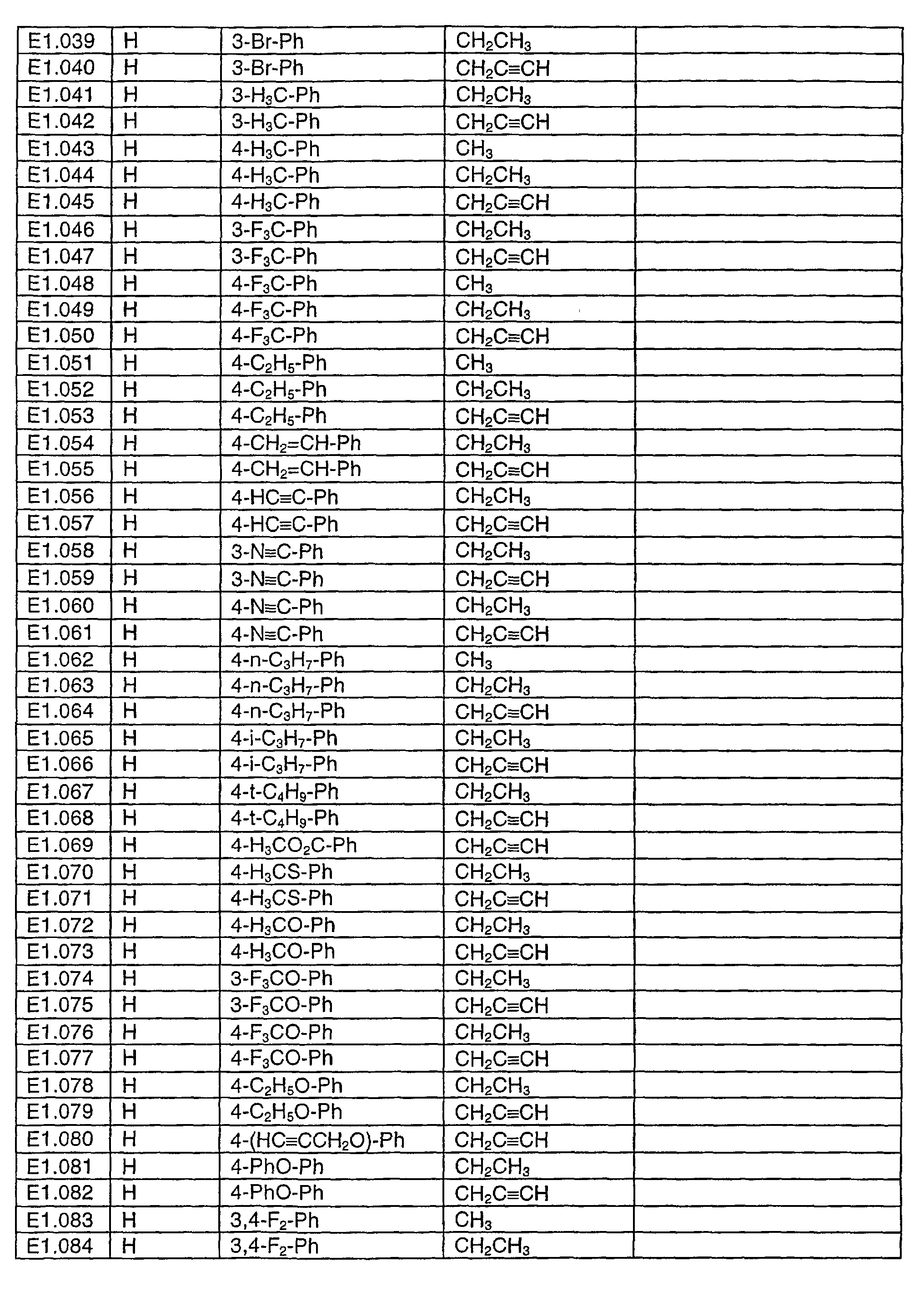

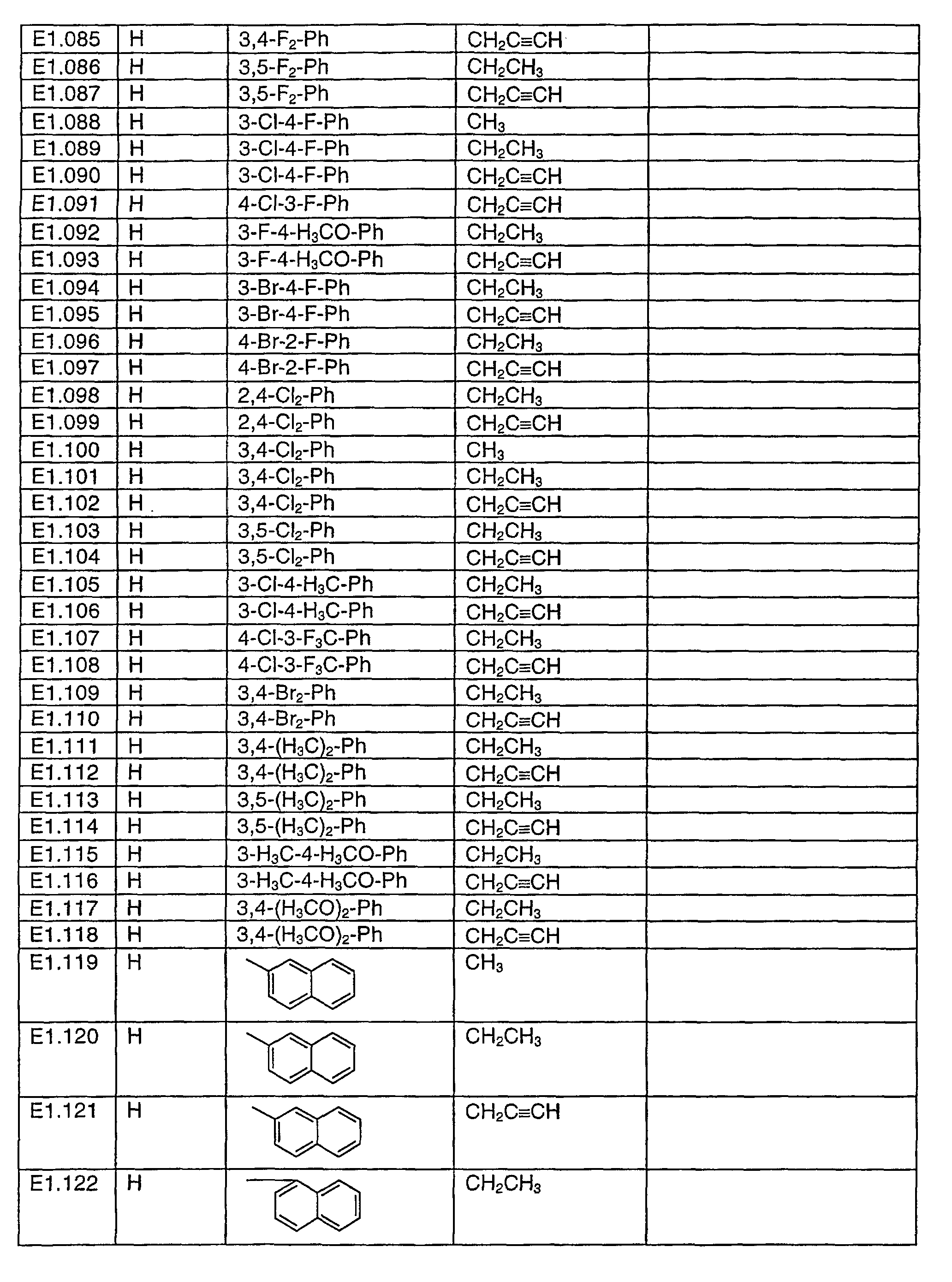

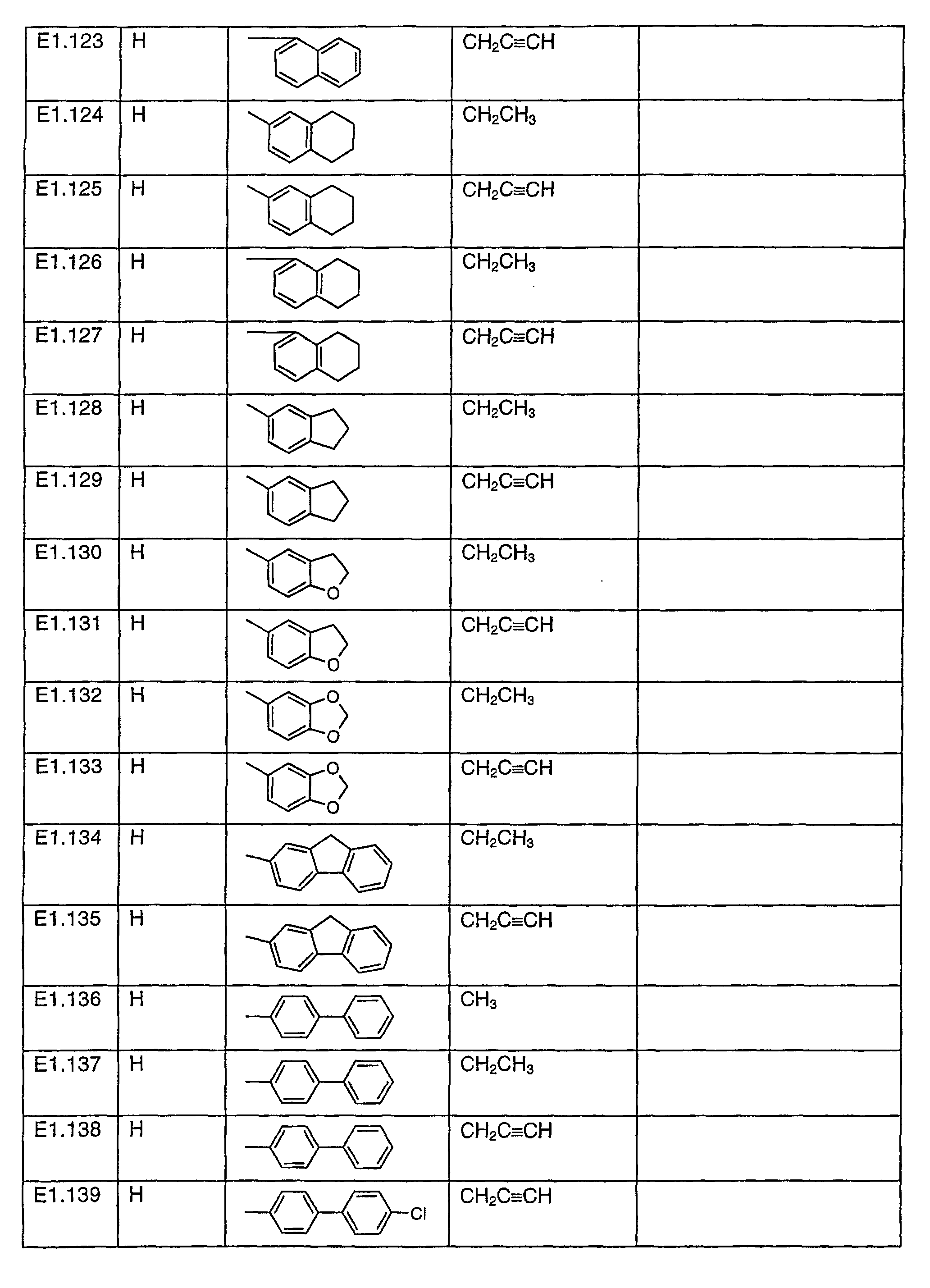

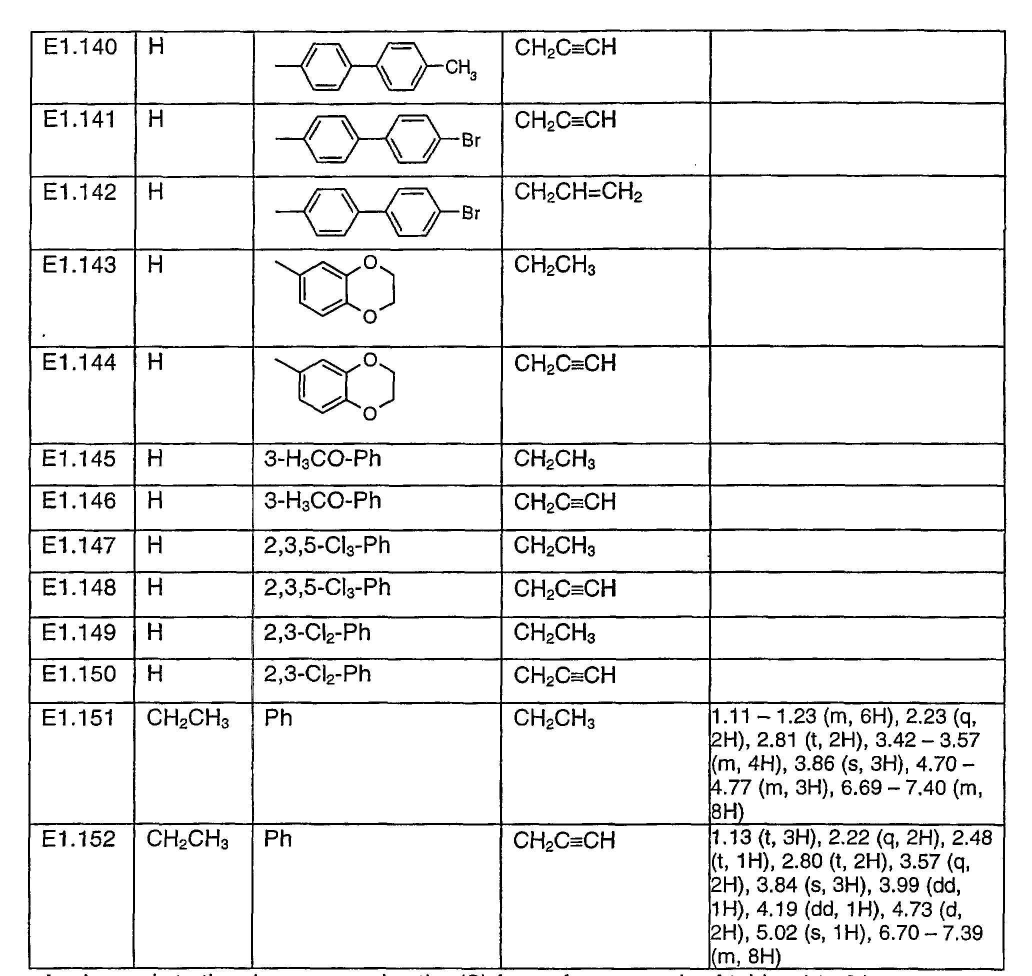

Analogously to the above examples the (S)-form of compounds of tables 1 to 31 are obtained.
Table 1: Compounds represented by the Formula I.01 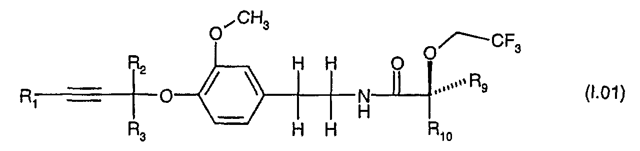 wherein the combination of the groups R₁, R₂, R₃, R₉ and R₁₀ corresponds each to one row in table A.
Table 2: Compounds represented by the Formula I.02 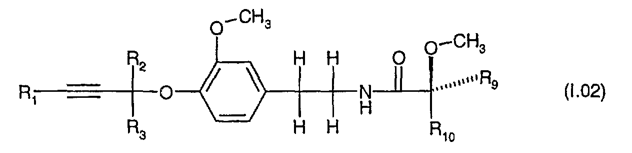 wherein the combination of the groups R₁, R₂, R₃, R₉ and R₁₀ corresponds each to one row in table A.
Table 3: Compounds represented by the Formula I.03 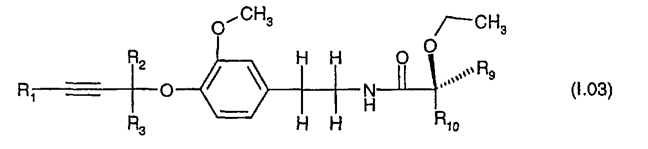 wherein the combination of the groups R₁, R₂, R₃, R₉ and R₁₀ corresponds each to one row in table A.
Table 4: Compounds represented by the Formula I.04 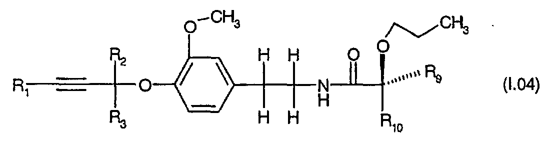 wherein the combination of the groups R₁, R₂, R₃, R₉ and R₁₀ corresponds each to one row in table A.
Table 5: Compounds represented by the Formula I.05 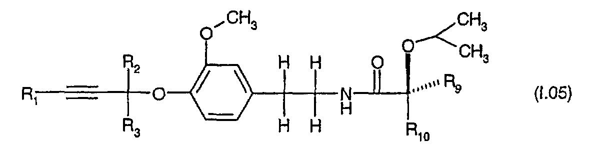 wherein the combination of the groups R₁, R₂, R₃, R₉ and R₁₀ corresponds to each one row in table A.
Table 6: Compounds represented by the Formula I.06 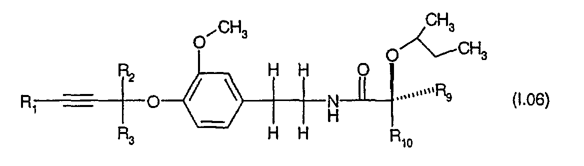 wherein the combination of the groups R₁, R₂, R₃, R₉ and R₁₀ corresponds each to one row in table A.
Table 7: Compounds represented by the Formula I.07 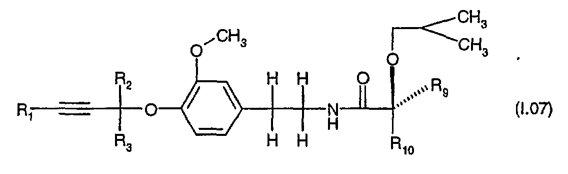 wherein the combination of the groups R₁, R₂, R₃, R₉ and R₁₀ corresponds each to one row in table A.
Table 8: Compounds represented by the Formula I.08 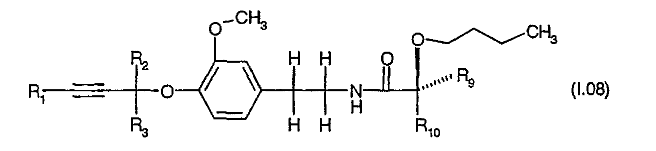 wherein the combination of the groups R₁, R₂, R₃, R₉ and R₁₀ corresponds each to one row in table A.
Table 9: Compounds represented by the Formula I.09 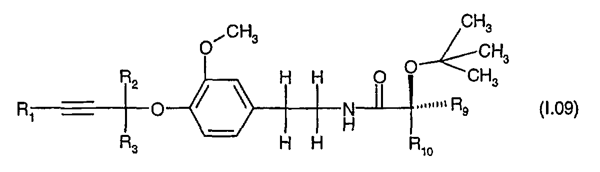 wherein the combination of the groups R₁, R₂, R₃, R₉ and R₁₀ corresponds each to one row in table A
Table 10: Compounds represented by the Formula I.10 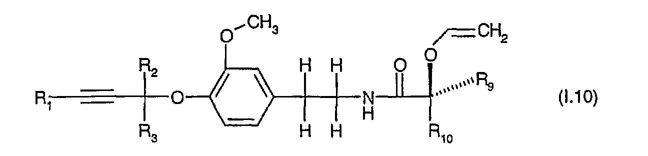 wherein the combination of the groups R₁, R₂, R₃, R₉ and R₁₀ corresponds each to one row in table A.
Table 11: Compounds represented by the Formula I.11 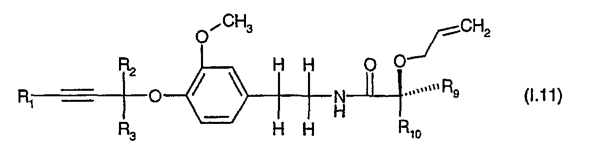 wherein the combination of the groups R₁, R₂, R₃, R₉ and R₁₀ corresponds each to one row in table A.
Table 12: Compounds represented by the Formula I.12 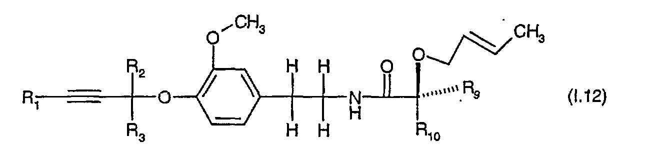 wherein the combination of the groups R₁, R₂, R₃, R₉ and R₁₀ corresponds each to one row in table A.
Table 13: Compounds represented by the Formula I.13 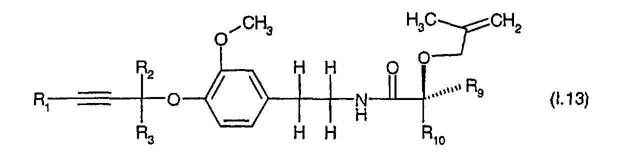 wherein the combination of the groups R₁, R₂, R₃, R₉ and R₁₀ corresponds each to one row in table A.
Table 14: Compounds represented by the Formula I.14 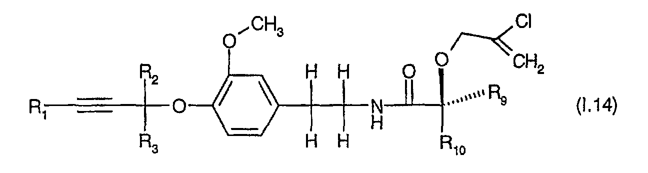 wherein the combination of the groups R₁, R₂, R₃, R₉ and R₁₀ corresponds each to one row in table A.
Table 15: Compounds represented by the Formula I.15 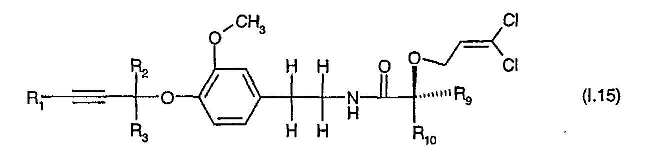 wherein the combination of the groups R₁, R₂, R₃, R₉ and R₁₀ corresponds each to one row in table A.
Table 16: Compounds represented by the Formula I.16 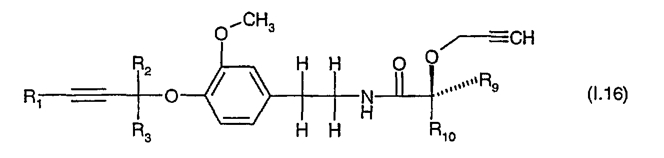 wherein the combination of the groups R₁, R₂, R₃, R₉ and R₁₀ corresponds each to one row in table A.
Table 17: Compounds represented by the Formula I.17 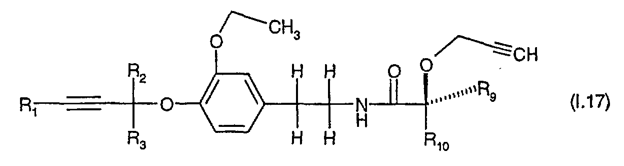 wherein the combination of the groups R₁, R₂, R₃, R₉ and R₁₀ corresponds each to one row in table A.
Table 18: Compounds represented by the Formula I.18 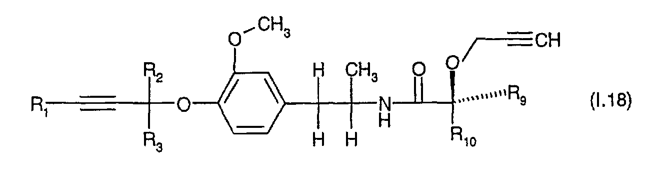 wherein the combination of the groups R₁, R₂, R₃, R₉ and R₁₀ corresponds each to one row in table A.
Table 19: Compounds represented by the Formula I.19 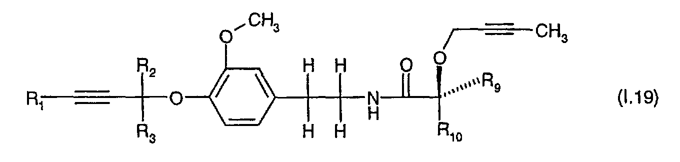 wherein the combination of the groups R₁, R₂, R₃, R₉ and R₁₀ corresponds each to one row in table A.
Table 20: Compounds represented by the Formula I.20 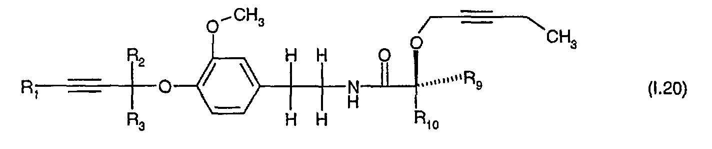 wherein the combination of the groups R₁, R₂, R₃, R₉ and R₁₀ corresponds each to one row in table A.
Table 21: Compounds represented by the Formula I.21 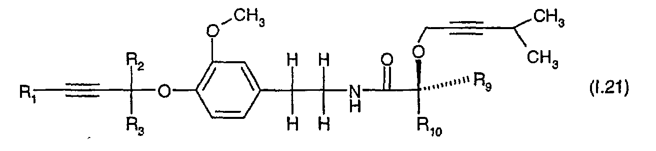 wherein the combination of the groups R₁, R₂, R₃, R₉ and R₁₀ corresponds each to one row in table A.
Table 22: Compounds represented by the Formula I.22 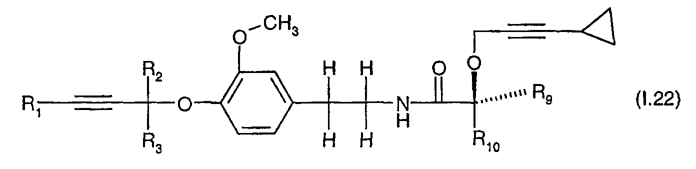 wherein the combination of the groups R₁, R₂, R₃, R₉ and R₁₀ corresponds each to one row in table A.
Table 23: Compounds represented by the Formula I.23 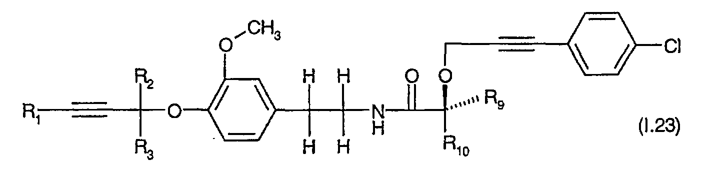 wherein the combination of the groups R₁, R₂, R₃, R₉ and R₁₀ corresponds each to one row in table A.
Table 24: Compounds represented by the Formula I.24 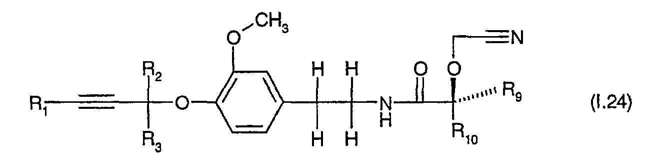 wherein the combination of the groups R₁, R₂, R₃, R₉ and R₁₀ corresponds each to one row in table A.
Table 25: Compounds represented by the Formula I.25 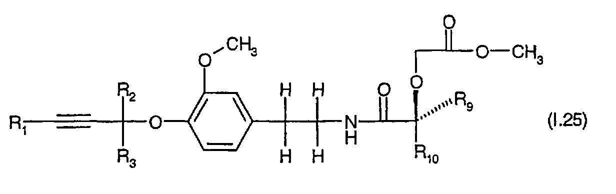 wherein the combination of the groups R₁, R₂, R₃, R₉ and R₁₀ corresponds each to one row in table A.
Table 26: Compounds represented by the Formula I.26 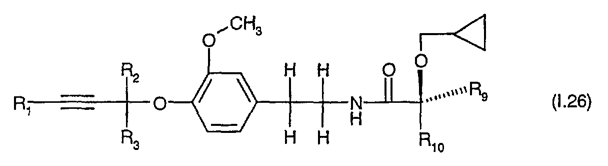 wherein the combination of the groups R₁, R₂, R₃, R₉ and R₁₀ corresponds each to one row in table A.
Table 27: Compounds represented by the Formula I.27 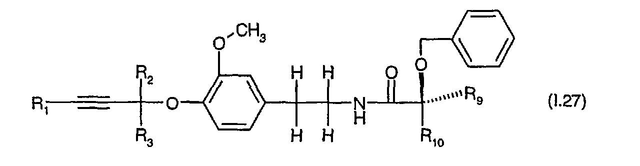 wherein the combination of the groups R₁, R₂, R₃, R₉ and R₁₀ corresponds each to one row in table A.
Table 28: Compounds represented by the Formula I.28 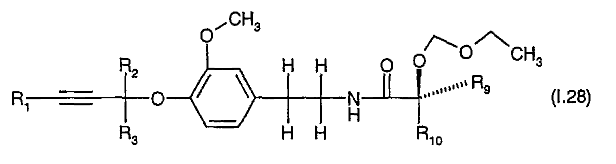 wherein the combination of the groups R₁, R₂, R₃, R₉ and R₁₀ corresponds each to one row in table A.
Table 29: Compounds represented by the Formula I.29 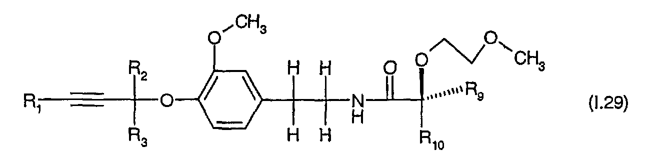 wherein the combination of the groups R₁, R₂, R₃, R₉ and R₁₀ corresponds each to one row in table A.
Table 30: Compounds represented by the Formula I.30 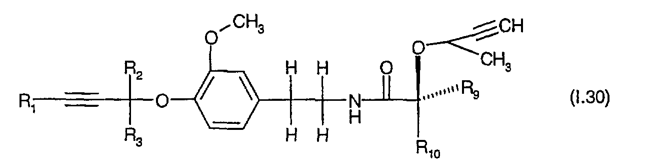 wherein the combination of the groups R₁, R₂, R₃, R₉ and R₁₀ corresponds each to one row in table A.
Table 31: Compounds represented by the Formula I.31 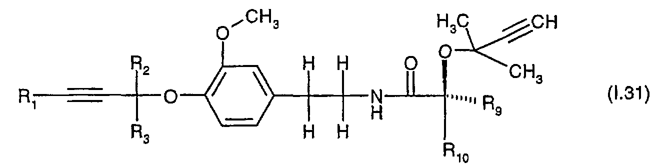 wherein the combination of the groups R₁, R₂, R₃, R₉ and R₁₀ corresponds each to one row in table A.

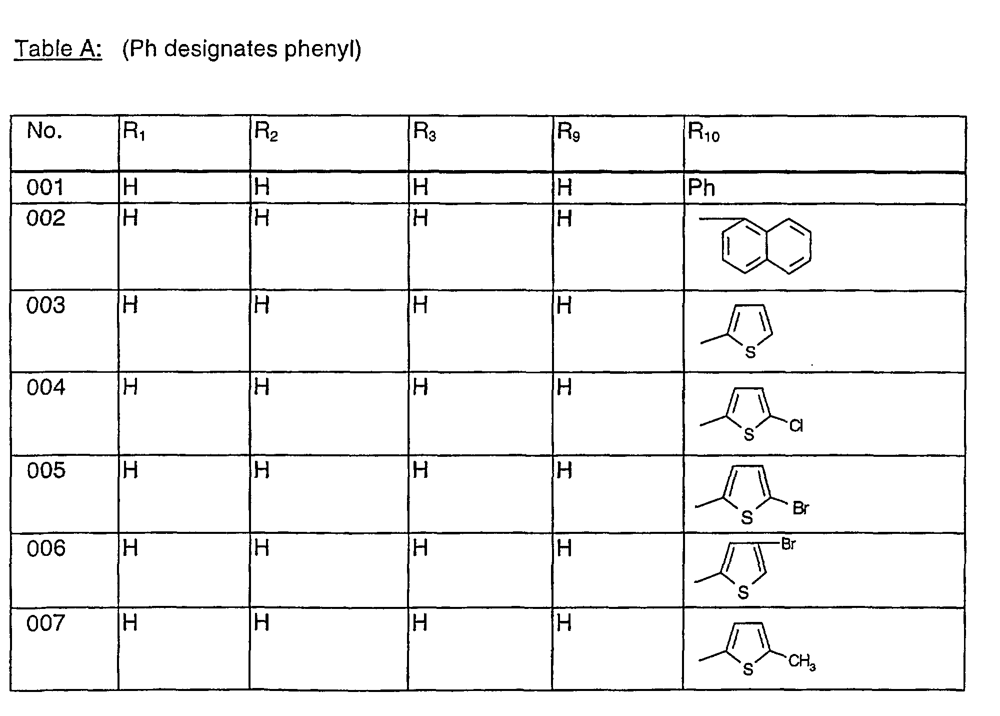

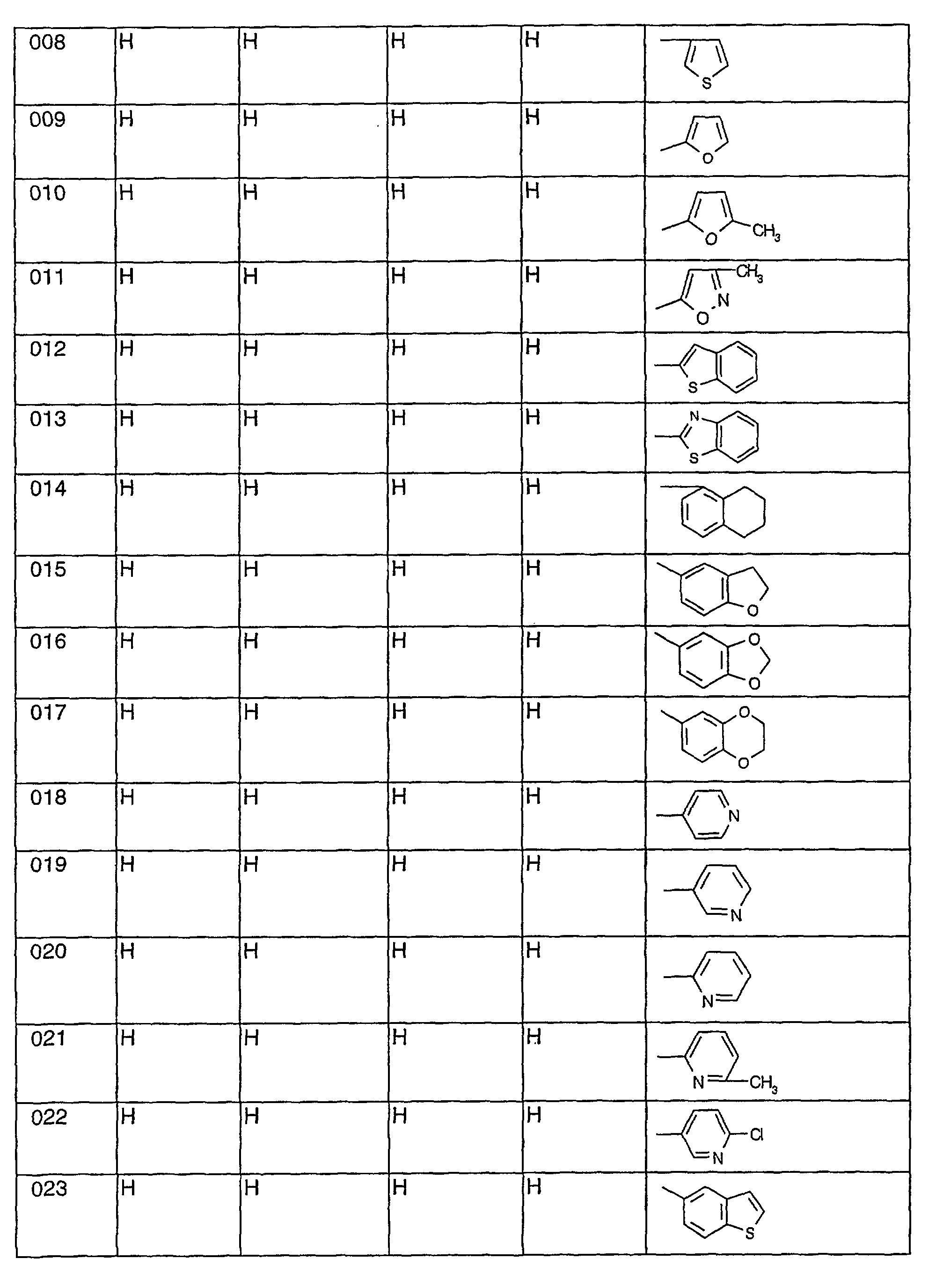

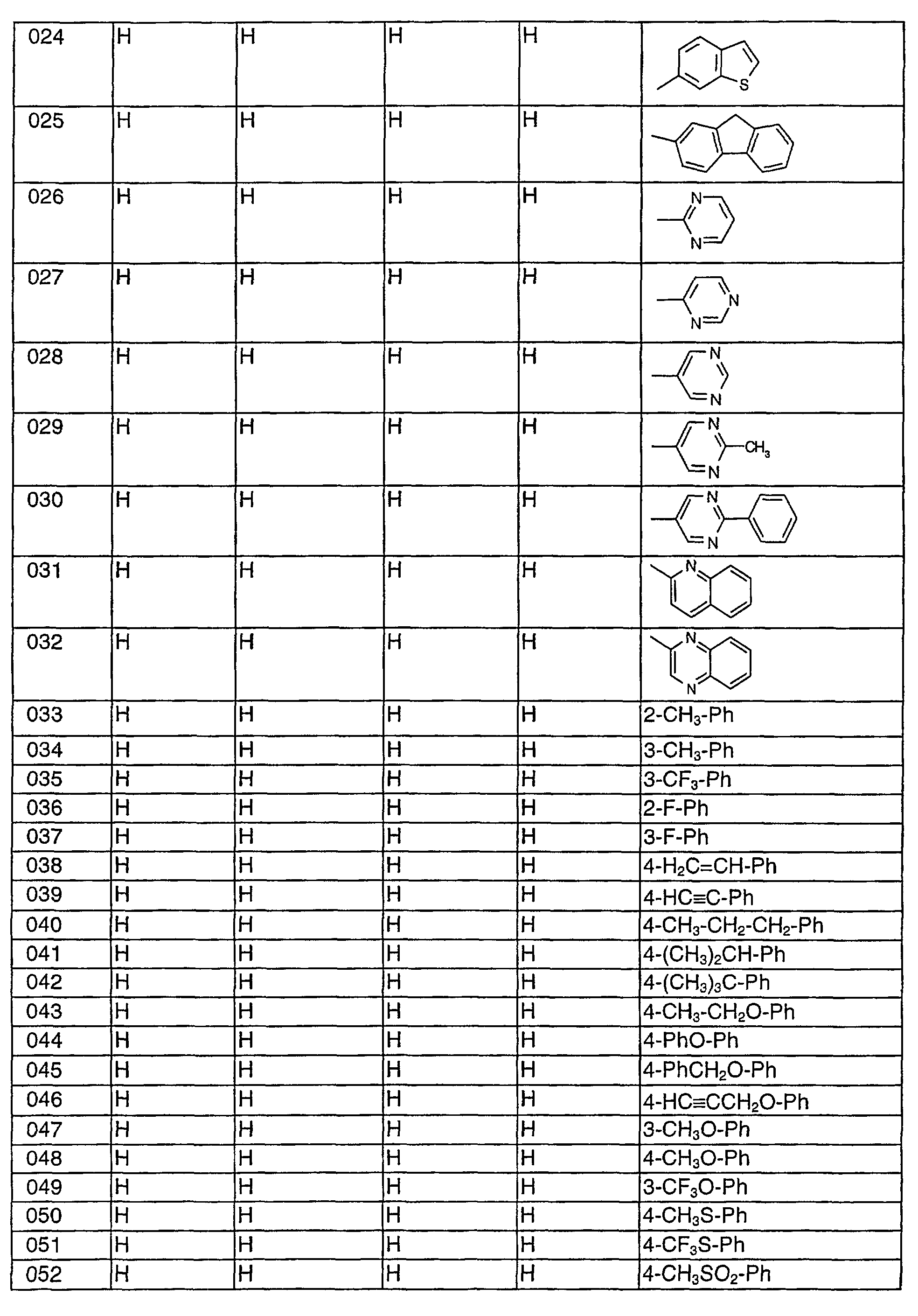

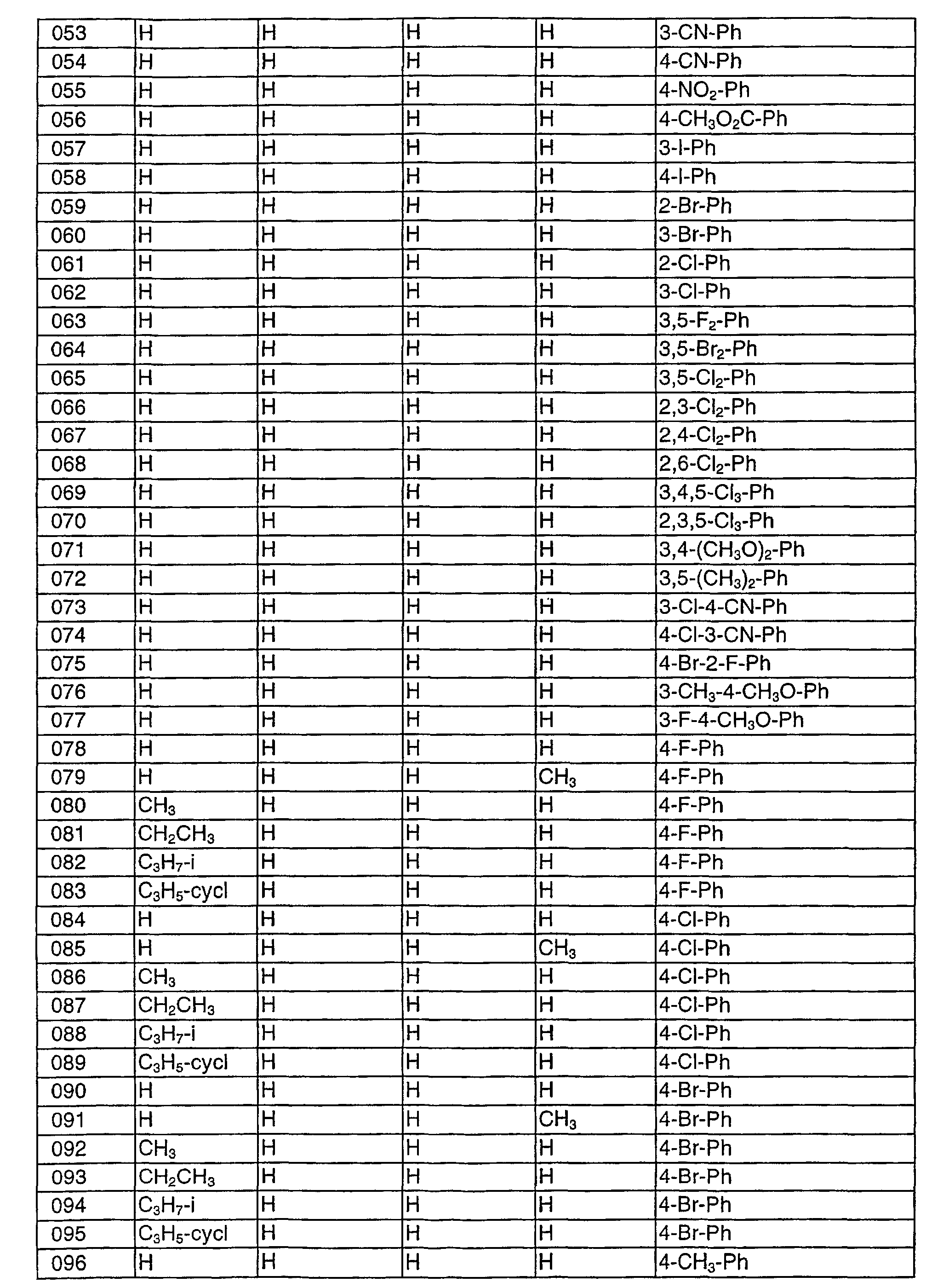

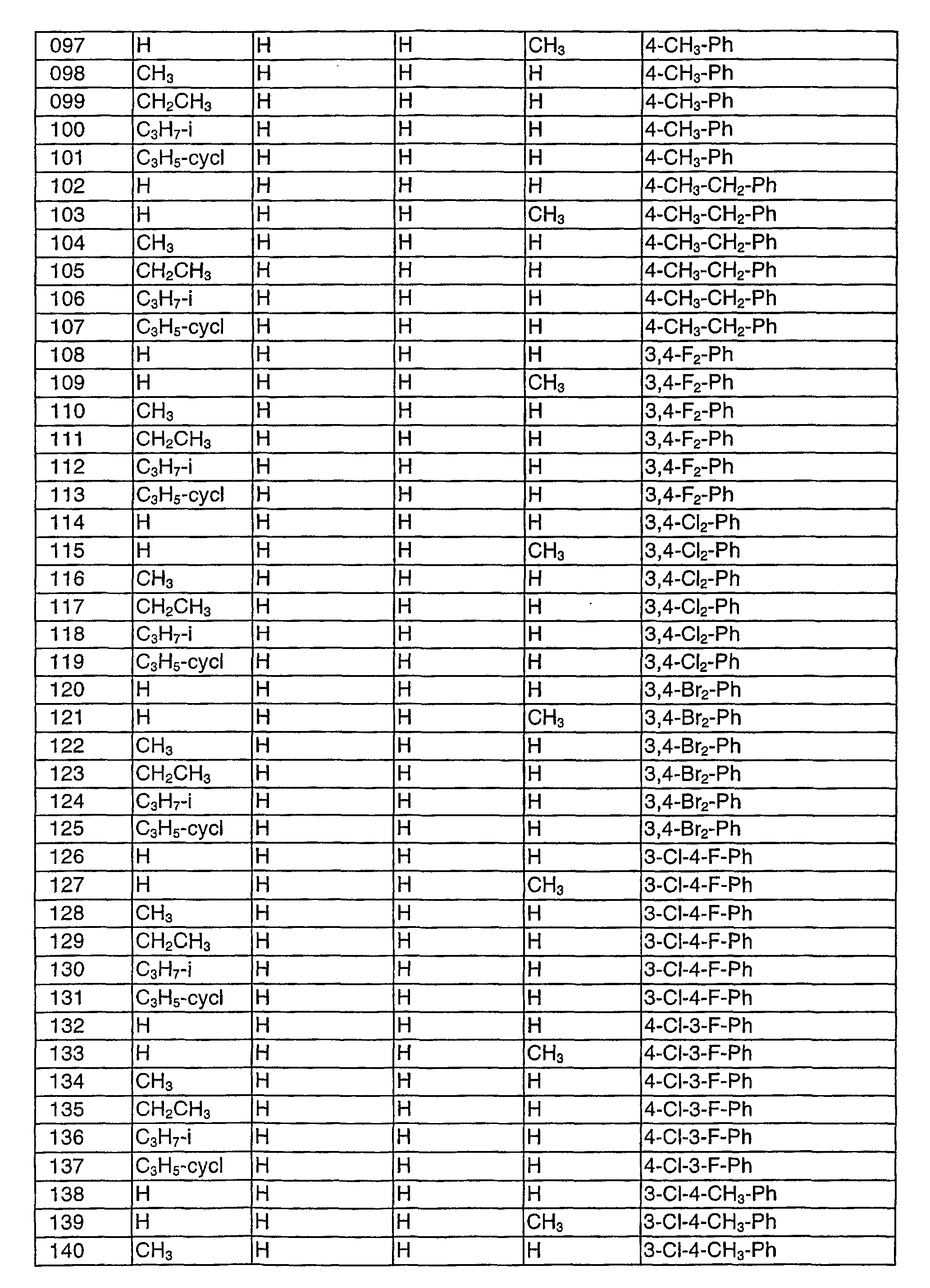

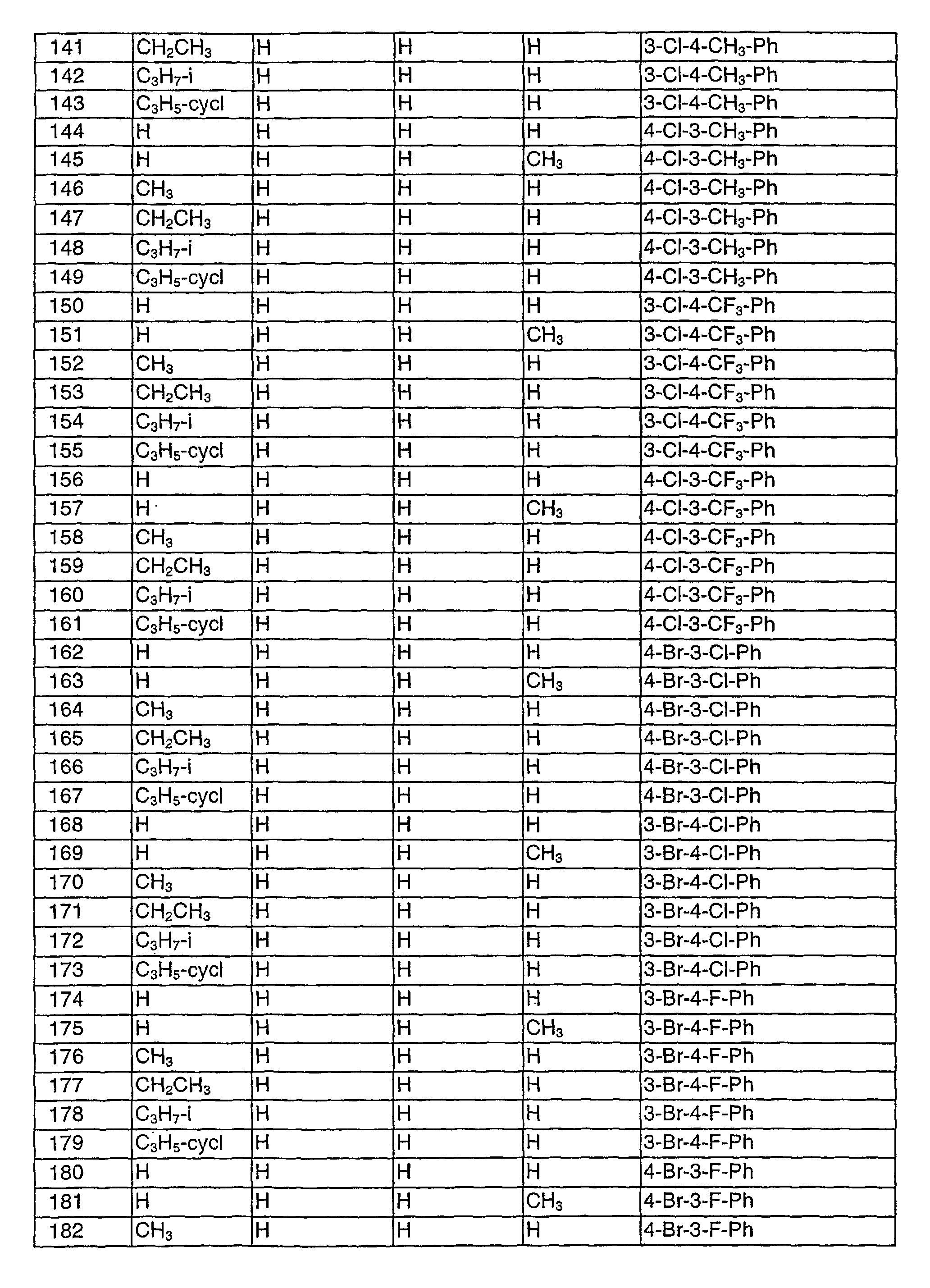

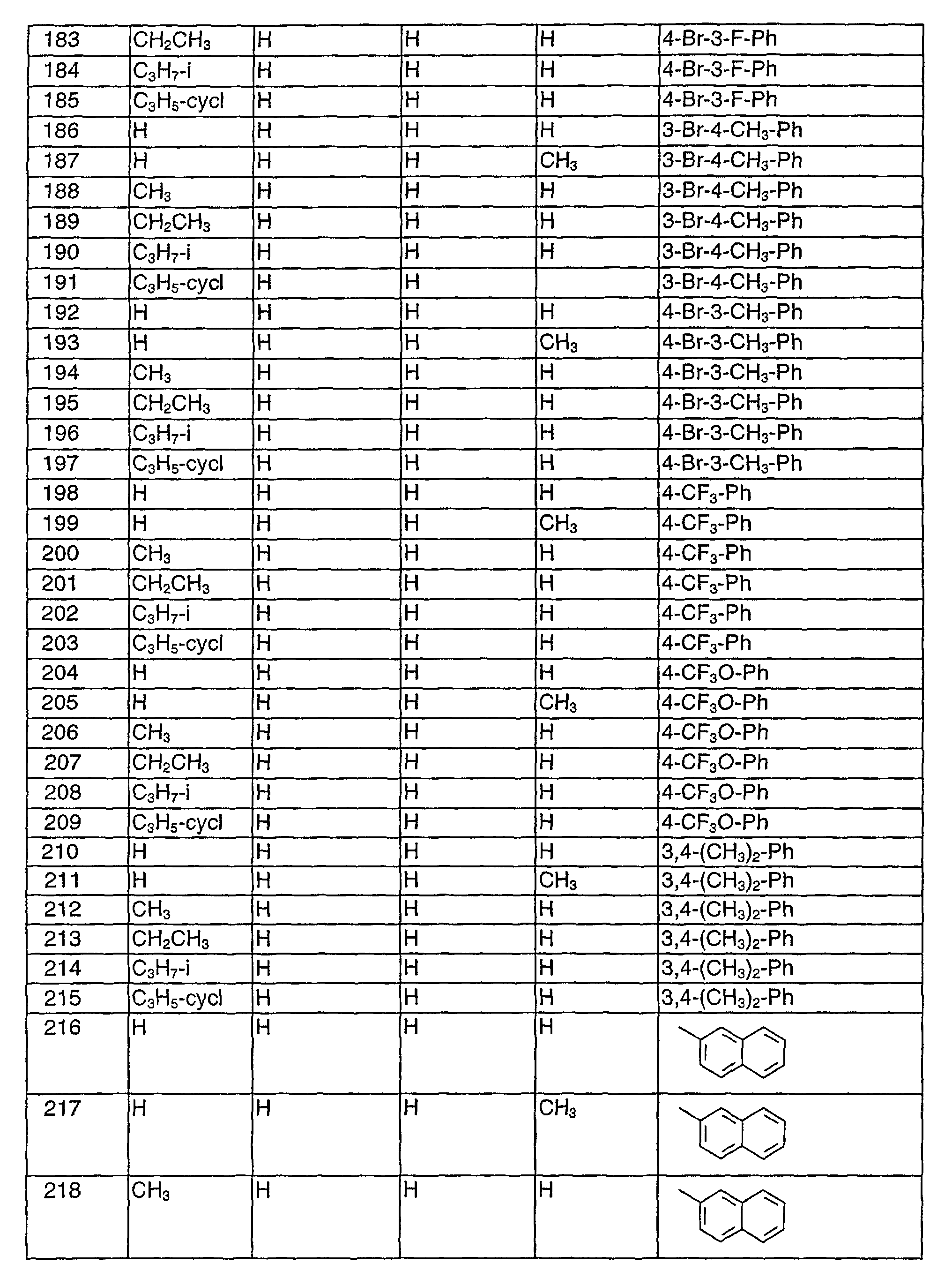

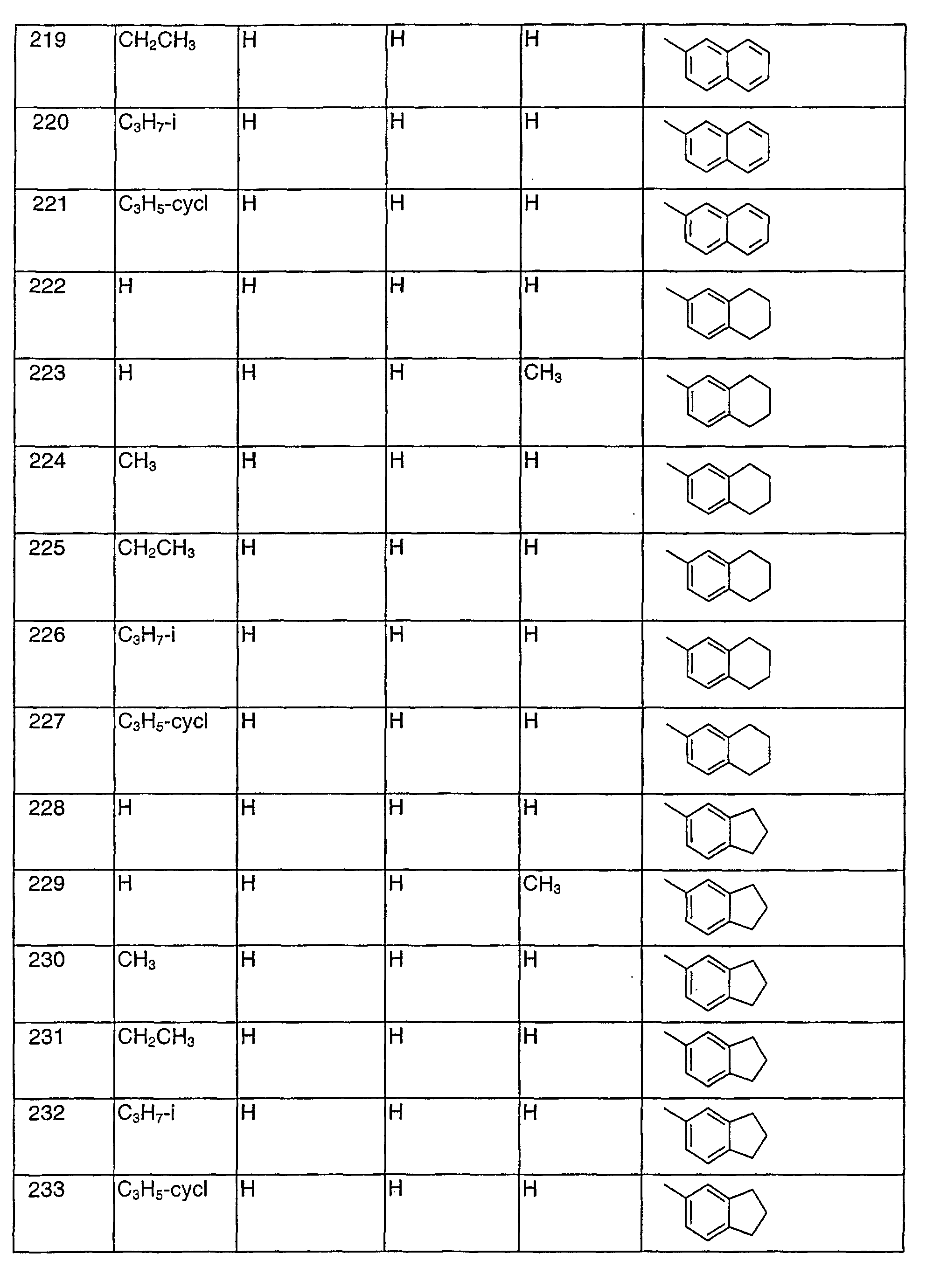

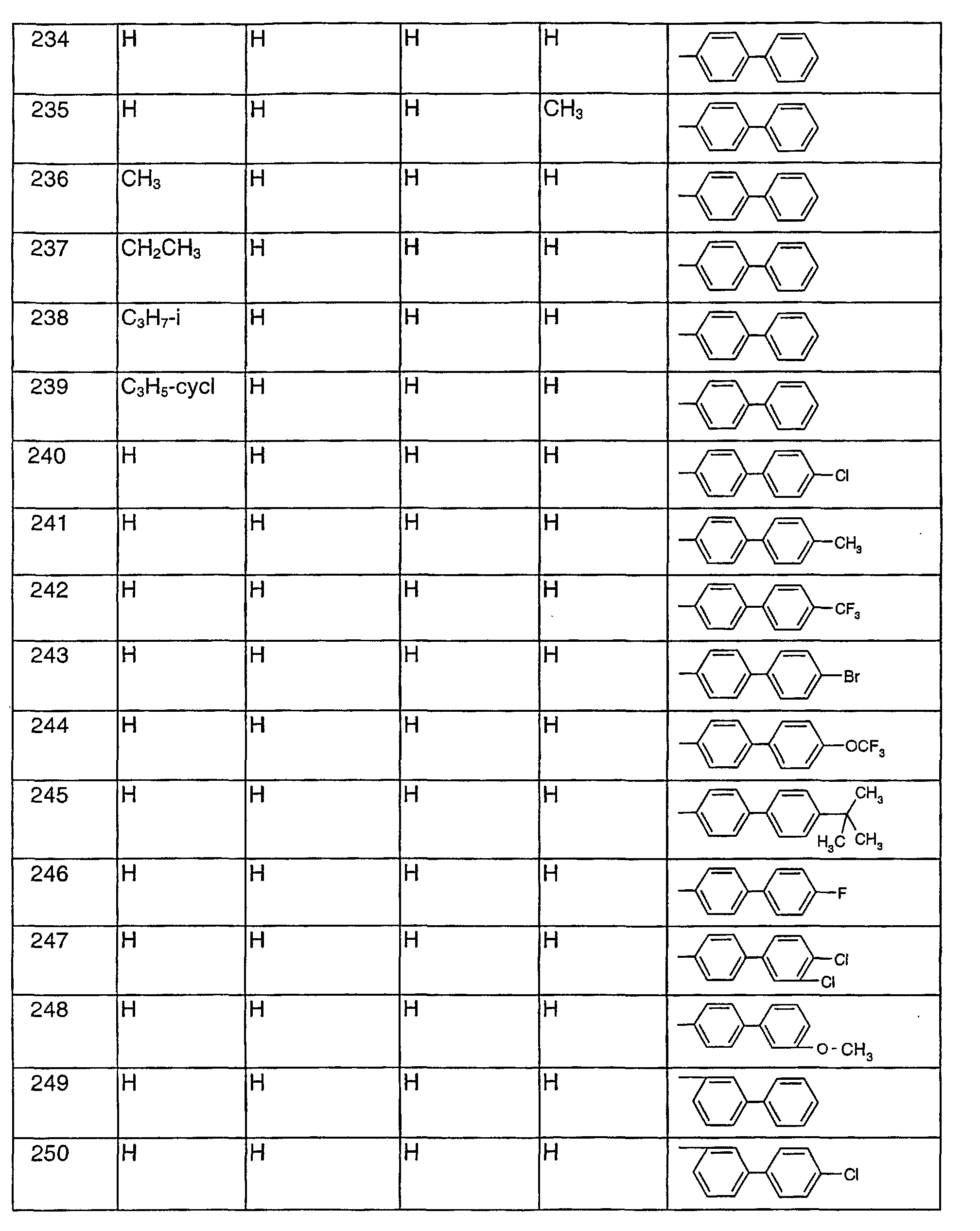

Formulations may be prepared analogously to those described in, for example, WO 95/30651.

### Biological Examples

### D-1: Action against Plasmopara viticola on vines

### a) Residual-protective action

Vine seedlings are sprayed at the 4- to 5-leaf stage with a spray mixture (0.02 % active ingredient) prepared from a wettable powder formulation of the test compound. After 24 hours, the treated plants are infected with a sporangia suspension of the fungus. Fungus infestation is evaluated after incubation for 6 days at 95-100 % relative humidity and +20°C.

### b) Residual-curative action

Vine seedlings are infected at the 4- to 5-leaf stage with a sporangia suspension of the fungus. After incubation for 24 hours in a humidity chamber at 95-100 % relative humidity and +20°C, the infected plants are dried and sprayed with a spray mixture (0.02 % active ingredient) prepared from a wettable powder formulation of the test compound. After the spray coating has dried, the treated plants are placed in the humidity chamber again. Fungus infestation is evaluated 6 days after infection.

Compounds of Tables 1 to 31 exhibit a good fungicidal action against Plasmopara viticola on vines. Compounds E1.002, E1.011 and E1.152 at 200 ppm inhibit fungal infestations in both tests D-1a) and D-1 b) by 80-100%. At the same time untreated plants showed pathogen attack of 80-100%.

### D-2: Action against Phytophthora on tomato plants

### a) Residual-protective action

After a cultivation period of 3 weeks, tomato plants are sprayed with a spray mixture (0.02 % active ingredient) prepared from a wettable powder formulation of the test compound. After 48 hours, the treated plants are infected with a sporangia suspension of the fungus. Fungus infestation is evaluated after incubation of the infected plants for 5 days at 90-100 % relative humidity and +20°C.

### b) Systemic action

After a cultivation period of 3 weeks, tomato plants are watered with a spray mixture (0.02 % active ingredient based on the volume of the soil) prepared from a wettable powder formulation of the test compound. Care is taken that the spray mixture does not come into contact with the parts of the plants that are above the ground. After 96 hours, the treated plants are infected with a sporangia suspension of the fungus. Fungus infestation is evaluated after incubation of the infected plants for 4 days at 90-100 % relative humidity and +20°C.

Compounds of Tables 1 to 31 exhibit a long-lasting effect against fungus infestation. Compounds E1.002, E1.011, E1.151 and E1.152 at 200 ppm inhibit fungal infestations in both tests D-2a) and D-2b) by 80 - 100 %. At the same time untreated plants showed pathogen attack of 80 - 100 %.

### D-3 : Action against Phytophthora on potato plants

### a) Residual-protective action

2-3 week old potato plants (Bintje variety) are sprayed with a spray mixture (0.02 % active ingredient) prepared from a wettable powder formulation of the test compound. After 48 hours, the treated plants are infected with a sporangia suspension of the fungus. Fungus infestation is evaluated after incubation of the infected plants for 4 days at 90-100 % relative humidity and +20°C.

### b) Systemic action

2-3 week old potato plants (Bintje variety) are watered with a spray mixture (0.02 % active ingredient based on the volume of the soil) prepared from a wettable powder formulation of the test compound. Care is taken that the spray mixture does not come into contact with the parts of the plants that are above the ground. After 48 hours, the treated plants are infected with a sporangia suspension of the fungus. Fungus infestation is evaluated after incubation of the infected plants for 4 days at 90-100 % relative humidity and +20°C. Fungal infestation is effectively controlled with compounds of Tables 1 to 31.

Compounds E1.002, E1.011 and E1.152 at 200 ppm inhibit fungal infestations in both tests D-3a) and D-3b) by 80 - 100 %. At the same time untreated plants showed pathogen attack of 80 - 100 %.

## Claims

1. The enantio-enriched compounds of formula I 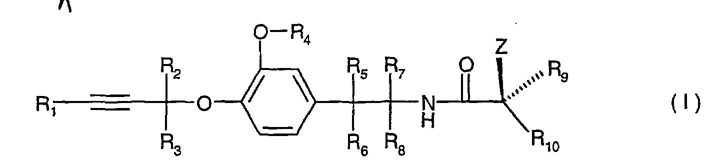 including the optical isomers thereof and mixtures of such isomers, wherein
R₁ is hydrogen, alkyl, cycloalkyl or optionally substituted aryl,
R₂ and R₃ are each independently hydrogen or alkyl,
R₄ is alkyl, alkenyl or alkynyl,
R₅, R₆, R₇, and R₈ are each independently hydrogen or alkyl,
R₉ is hydrogen, optionally substituted alkyl, optionally substituted alkenyl or optionally substituted alkynyl,
R₁₀ is optionally substituted aryl or optionally substituted heteroaryl, and
Z is optionally substituted aryloxy, optionally substituted alkoxy, optionally substituted alkenyloxy or optionally substituted alkynyloxy

2. A compound according to claim 1 wherein the enantio-enriched form of the compound of formula I contains the pure enantiomer as displayed in an amount of at least 70%.

3. A compound according to claims 1 or 2 wherein the contents of the entio-enriched form contains the pure enantiomer as displayed in an amount of at least 80%, preferably above 90%, especially above 95%.

4. A compound according to any one of claims 1 to 3, wherein
R₁ is hydrogen, alkyl, cycloalkyl, phenyl or naphthyl; phenyl and naphthyl being optionally substituted by substituents selected from the group comprising alkyl, alkenyl, alkynyl, cycloalkyl, cycloalkyl-alkyl, phenyl and phenylalkyl, where all these groups may in turn be substituted by one or several halogens; alkoxy, alkenyloxy, alkynyloxy; alkoxy-alkyl; haloalkoxy; alkylthio; haloalkylthio; alkylsulfonyl; formyl; alkanoyl; hydroxy; halogen; cyano; nitro; amino; alkylamino; dialkylamino; carboxyl; alkoxycarbonyl; alkenyloxycarbonyl; or alkynyloxycarbonyl; and
R₂ and R₃ are independently of each other hydrogen or C₁-C₄alkyl; and
R₄ is C₁-C₈alkyl, C₂-C₈alkenyl, or C₂-C₈alkynyl; and
R₅, R₆, R₇ and R₈ are independently of each other hydrogen or C₁-C₄alkyl; and
R₉ is hydrogen, C₁-C₄alkyl, C₁-C₄haloalkyl, C₃-C₄alkenyl or C₃-C₄alkynyl; and
R₁₀ is aryl or heteroaryl, each optionally substituted with substituents selected from to group comprising alkyl, alkenyl, alkynyl, cycloalkyl, cycloalkyl-alkyl, phenyl and phenylalkyl, where all these groups may be substituted with one or more substituents selected from the group comprising halogen; alkoxy, alkenyloxy, alkynyloxy; alkoxy-alkyl; haloalkoxy; alkylthio; haloalkylthio; alkylsulfonyl; formyl; alkanoyl; hydroxy; cyano; nitro; amino; alkylamino; dialkylamino; carboxyl; alkoxycarbonyl; alkenyloxycarbonyl and alkynyloxycarbonyl; and
Z is optionally substituted aryloxy wherein the aryl may be optionally substituted by one or more substituents selected from the group comprising halogen, C₁-C₈alkoxy, C₂-C₈alkenyloxy, C₂-C₈alkynyloxy, C₁-C₈alkoxy-C₁-C₈alkyl, C₁-C₈haloalkoxy, C₁-C₈alkylthio, C₁-C₈haloalkylthio, C₁-C₈alkylsulfonyl, formyl, C₂-C₈alkanoyl, hydroxy, halogen, cyano, nitro, amino, C₁-C₈alkylamino, di-C₁-C₈alkylamino, carboxyl and C₁-C₈alkoxycarbonyl; or is optionally substituted C₁-C₈alkoxy, optionally substituted C₂-C₈alkenyloxy or optionally substituted C₂-C₈alkynyloxy, wherein each alkyl, alkenyl or alkynyl group may carry one or more substituents selected from the group comprising halogen, C₁-C₄alkyl, C₁-C₄alkoxy, C₁-C₄alkylthio, C₃-C₆cycloalkyl, nitro, cyano, hydroxy, phenyl, mercapto, C₁-C₄alkylcarbonyl and C₁-C₄alkoxycarbonyl.

5. A compound according to any one of claims 1 to 4 wherein R₁ is hydrogen, C₁-C₈alkyl, C₃-C₈cycloalkyl, phenyl or naphthyl; phenyl and naphthyl being optionally substituted by one to three substituents selected from the group comprising C₁-C₈alkyl, C₂-C₈alkenyl, C₂-C₈alkynyl, C₁-C₈haloalkyl, C₁-C₈alkoxy, C₁-C₈haloalkoxy, C₁-C₈alkylthio, C₁-C₈haloalkylthio, C₁-C₈alkylsulfonyl, halogen, cyano, nitro and C₁-C₈alkoxycarbonyl; and R₂ and R₃ are hydrogen; and R₄ is C₁-C₆alkyl; and R₅, R₆ and R₇ are hydrogen and R₈ is hydrogen, methyl or ethyl; and R₉ is hydrogen or C₁-C₄alkyl; and R₁₀ is phenyl, naphthyl or biphenyl, each optionally substituted by one to three substituents selected from the group comprising C₁-C₈alkyl, C₂-C₈alkenyl, C₂-C₈alkynyl, C₁-C₈haloalkyl, C₁-C₈alkoxy, C₁-C₈haloalkoxy, C₁-C₈alkylthio, C₁-C₈haloalkylthio, C₁-C₈alkylsulfonyl, halogen, cyano, nitro and C₁-C₈alkoxycarbonyl; and Z is C₁-C₈alkoxy, C₂-C₈alkenyloxy, C₁-C₈alkynyloxy, C₁-C₈alkoxy-C₁-C₈alkoxy, C₂-C₈alkenyloxy-C₁-C₈alkoxy, C₂-C₈alkynyloxy-C₁-C₈alkoxy, C₁-C₈haloalkoxy or C₃-C₈cycloalkyl-C₁-C₈alkoxy.

6. A compound of formula I according to any one of claims 1 to 5 wherein R₁ is hydrogen, C₁-C₈alkyl or phenyl optionally substituted by one to three substituents selected from the group comprising C₁-C₈alkyl, C₁-C₈haloalkyl, C₁-C₈alkoxy, C₁-C₈haloalkoxy, C₁-C₈alkylthio, C₁-C₈haloalkylthio, halogen, cyano, nitro and C₁-C₈alkoxycarbonyl; and R₂ and R₃ are hydrogen; and R₄ is C₁-C₄alkyl, and R₅, R₆ and R₇ are hydrogen and R₈ is hydrogen or methyl; and R₉ is hydrogen; and R₁₀ is phenyl, naphthyl, 1,3-biphenyl or 1,4-biphenyl, each optionally substituted by one to three substituents selected from the group comprising C₁-C₈alkyl, C₁-C₈haloalkyl, C₁-C₈alkoxy, C₁-C₈haloalkoxy, C₁-C₈alkylthio, C₁-C₈haloalkylthio, halogen, cyano, nitro and C₁-C₈alkoxycarbonyl; and Z is C₁-C₈alkoxy, C₂-C₈alkenyloxy, C₂-C₈alk-ynyloxy or C₁-C₄alkoxy-C₁-C₂alkoxy.

7. A compound of formula I according to any on of claims 1 to 6 wherein R₁ is hydrogen, C₁-C₈alkyl or C₃-C₈cycloalkyl; and R₂, R₃, R₅, R₆, R₇, R₈ and R₉ are hydrogen; and R₄ is methyl or ethyl; and R₁₀ is phenyl, naphthyl, 1,3-biphenyl or 1,4-biphenyl, each optionally substituted by one to three substituents selected from the group comprising C₁-C₈alkyl, C₁-C₈haloalkyl, C₁-C₈alkoxy, C₁-C₈haloalkoxy, C₁-C₈alkylthio, C₁-C₈haloalkylthio, halogen, cyano, nitro and C₁-C₈alkoxycarbonyl.

8. A compound of formula I according to claim 1 selected from the group comprising
(S)-2-(4-bromo-phenyl)-2-methoxy-N-[2-(3-methoxy-4-prop-2-ynyloxy-phenyl)-ethyl]-acetamide,
(S)-2-(4-chloro-phenyl)-2-methoxy-N-[2-(3-methoxy-4-prop-2-ynyloxy-phenyl)-ethyl]-acetamide,
(S)-2-(3,4-dichloro-phenyl)-2-methoxy-N-[2-(3-methoxy-4-prop-2-ynyloxy-phenyl)-ethyl]-acetamide,
(S)-2-(4-bromo-phenyl)-2-ethoxy-N-[2-(3-methoxy-4-prop-2-ynyloxy-phenyl)-ethyl]-acetamide,
(S)-2-(4-chloro-phenyl)-2-ethoxy-N-[2-(3-methoxy-4-prop-2-ynyloxy-phenyl)-ethyl]-acetamide,
(S)-2-(3,4-dichloro-phenyl)-2-ethoxy-N-[2-(3-methoxy-4-prop-2-ynyloxy-phenyl)-ethyl]-acetamide,
(S)-2-(4-bromo-phenyl)-N-[2-(3-methoxy-4-prop-2-ynyloxy-phenyl)-ethyl]-2-propoxy-acetamide,
(S)-2-(4-chloro-phenyl)-N-[2-(3-methoxy-4-prop-2-ynyloxy-phenyl)-ethyl]-2-propoxy-acetamide,
(S)-2-(3,4-dichloro-phenyl)-N-[2-(3-methoxy-4-prop-2-ynyloxy-phenyl)-ethyl]-2-propoxy-acetamide,
(S)-2-(4-bromo-phenyl)-2-cyclopropylmethoxy-N-[2-(3-methoxy-4-prop-2-ynyloxy-phenyl)-ethyl]-acetamide,
(S)-2-(4-chloro-phenyl)-2-cyclopropylmethoxy-N-[2-(3-methoxy-4-prop-2-ynyloxy-phenyl)-ethyl]-acetamide,
(S)-2-cyclopropylmethoxy-2-(3,4-dichloro-phenyl)-N-[2-(3-methoxy-4-prop-2-ynyloxy-phenyl)-ethyl]-acetamide,
(S)-2-(4-bromo-phenyl)-2-ethoxymethoxy-N-[2-(3-methoxy-4-prop-2-ynyloxy-phenyl)-ethyl]-acetamide,
(S)-2-(4-chloro-phenyl)-2-ethoxymethoxy-N-[2-(3-methoxy-4-prop-2-ynyloxy-phenyl)-ethyl]-acetamide,
(S)-2-(3,4-dichloro-phenyl)-2-ethoxymethoxy-N-[2-(3-methoxy-4-prop-2-ynyloxy-phenyl)-ethyl]-acetamide,
(S)-2-allyloxy-2-(4-bromo-phenyl)-N-[2-(3-methoxy-4-prop-2-ynyloxy-phenyl)-ethyl]-acetamide,
(S)-2-allyloxy-2-(4-chloro-phenyl)-N-[2-(3-methoxy-4-prop-2-ynyloxy-phenyl)-ethyl]-acetamide,
(S)-2-allyloxy-2-(3,4-dichloro-phenyl)-N-[2-(3-methoxy-4-prop-2-ynyloxy-phenyl)-ethyl]-acetamide,
(S)-2-(4-bromo-phenyl)-2-(but-2-enyloxy)-N-[2-(3-methoxy-4-prop-2-ynyloxy-phenyl)-ethyl]-acetamide,
(S)-2-(but-2-enyloxy)-2-(4-chloro-phenyl)-N-[2-(3-methoxy-4-prop-2-ynyloxy-phenyl)-ethyl]-acetamide,
(S)-2-(but-2-enyloxy)-2-(3,4-dichloro-phenyl)-N-[2-(3-methoxy-4-prop-2-ynyloxy-phenyl)-ethyl]-acetamide,
(S)-2-(4-bromo-phenyl)-N-[2-(3-methoxy-4-prop-2-ynyloxy-phenyl)-ethyl]-2-prop-2-ynyloxy-acetamide,
(S)-2-(4-chloro-phenyl)-N-[2-(3-methoxy-4-prop-2-ynyloxy-phenyl)-ethyl]-2-prop-2-ynyloxy-acetamide,
(S)-2-(3,4-dichloro-phenyl)-N-[2-(3-methoxy-4-prop-2-ynyloxy-phenyl)-ethyl]-2-prop-2-ynyloxy-acetamide,
(S)-2-biphenyl-4-yl-N-[2-(3-methoxy-4-prop-2-ynyloxy-phenyl)-ethyl]-2-prop-2-ynyloxy-acetamide,
(S)-N-[2-(3-methoxy-4-prop-2-ynyloxy-phenyl)-ethyl]-2-naphthalen-2-yl-2-prop-2-ynyloxy-acetamide,
(S)-N-[2-(3-methoxy-4-prop-2-ynyloxy-phenyl)-ethyl]-2-prop-2-ynyloxy-2-*p*-tolyl-acetamide, 2-(4-ethyl-phenyl)-N-[2-(3-methoxy-4-prop-2-ynyloxy-phenyl)-ethyl]-2-prop-2-ynyloxy-acetamide,
(S)-N-[2-(3-methoxy-4-prop-2-ynyloxy-phenyl)-ethyl]-2-prop-2-ynyloxy-2-(4-trifluoromethyl-phenyl)-acetamide,
(S)-2-(4-bromo-phenyl)-2-but-2-ynyloxy-N-[2-(3-methoxy-4-prop-2-ynyloxy-phenyl)-ethyl]-acetamide,
(S)-2-but-2-ynyloxy-2-(4-chloro-phenyl)-N-[2-(3-methoxy-4-prop-2-ynyloxy-phenyl)-ethyl]-acetamide,
(S)-2-but-2-ynyloxy-2-(3,4-dichloro-phenyl)-N-[2-(3-methoxy-4-prop-2-ynyloxy-phenyl)-ethyl]-acetamide,
(S)-2-(4-bromo-phenyl)-N-[2-(3-methoxy-4-pent-2-ynyloxy-phenyl)-ethyl]-2-prop-2-ynyloxy-acetamide,
(S)-2-(4-chloro-phenyl)-N-[2-(3-methoxy-4-pent-2-ynyloxy-phenyl)-ethyl]-2-prop-2-ynyloxy-acetamide,
(S)-2-(3,4-dichloro-phenyl)-N-[2-(3-methoxy-4-pent-2-ynyloxy-phenyl)-ethyl]-2-prop-2-ynyloxy-acetamide,
(S)-2-(4-fluoro-phenyl)-N-[2-(3-methoxy-4-prop-2-ynyloxy-phenyl)-ethyl]-2-prop-2-ynyloxy-acetamide,
(S)-2-(3,4-difluoro-phenyl)-N-[2-(3-methoxy-4-prop-2-ynyloxy-phenyl)-ethyl]-2-prop-2-ynyloxy-acetamide,
(S)-2-(4-chloro-3-fluoro-phenyl)-N-[2-(3-methoxy-4-prop-2-ynyloxy-phenyl)-ethyl]-2-prop-2-ynyloxy-acetamide, and
(S)-2-(3-chloro-4-fluoro-phenyl)-N-[2-(3-methoxy-4-prop-2-ynyloxy-phenyl)-ethyl]-2-prop-2-ynyloxy-acetamide.

9. A process for the preparation of a compound of formula I according to claim 1, which comprises reacting
a) the phenol of formula IV 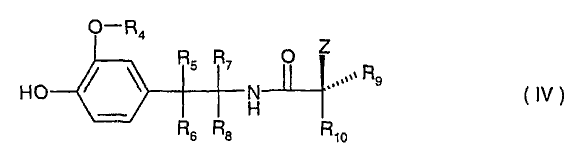 wherein R₄, R₅, R₆, R₇, R₈, R₉, R₁₀ and Z are as defined for formula I with a compound of formula V 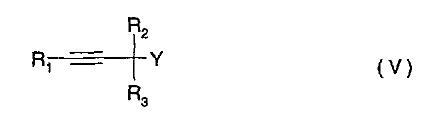 wherein R₁, R₂ and R₃ are as defined for formula I and wherein Y is a leaving group; or
b) the acid of formula II 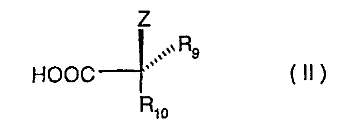 with an amine of formula VI 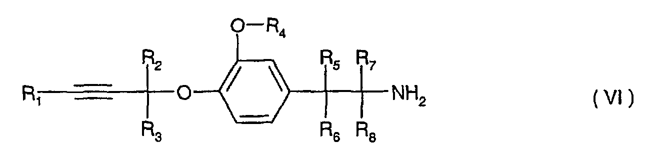
wherein R₁, R₂, R₃, R₄, R₅, R₆, R₇, R₈, R₉ and R₁₀ are as defined for formula I.

10. A composition for controlling and protecting against phytopathogenic microorganisms, comprising a compound of formula I according to claim 1 as active ingredient together with a suitable carrier.

11. A composition according to claim 10 which comprises at least one additional fungicidally active compound, preferably selected from the group consisting of cymoxanil, trifloxystrobin, azoxystrobin, picoxystrobin, chlorothalonil, metalaxyl, metalaxyl-M, pyraclostrobin (BAS500F), dimethomorph, fosetyl-Al, copper-salts, acibenzolar-S-methyl, fludioxonil, mancozeb, folpet, fluazinam, iprovalicarb, zoxamid and (S)-2-(methylsulfonyl-amino)-3-methyl-butyric acid N-[2-{3-methoxy-4-(3-(4-chlorophenyl)-2-propyn-1-yloxy)-phenyl}-ethyl]-amide.

12. The use of a compound of formula I according to claim 1 in protecting plants against infestation by phytopathogenic microorganisms.

13. A method of controlling and preventing an infestation of crop plants by phytopathogenic microorganisms, which comprises the application of a compound of formula I according to claim 1 as active ingredient to the plant, to parts of plants or to the locus thereof.

14. A method according to claim 13, wherein the phytopathogenic microorganisms are fungal organisms.

15. A process for the preparation of a compound of formula la according to claim 1 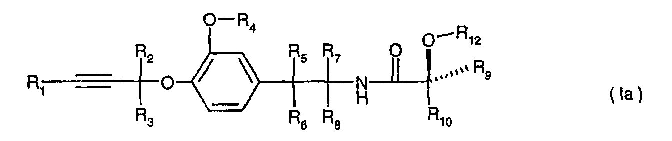 wherein R₁, R₂, R₃, R₄, R₅, R₆, R₇, R₈, R₉ and R₁₀ are as defined for formula I in claim 1 and R₁₂ is alkyl, alkenyl or alkynyl, which process comprises reacting
a compound of formula XIII 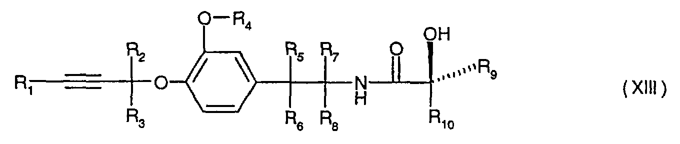 with a compound of formula XIV
Y-R₁₂ (XIV)
wherein R₁₂ is alkyl, alkenyl or alkynyl and Y is a leaving group, e.g. bromine or chlorine or a tosyl, mesyl or trifluoromethylsulfonyl.

16. A process for the preparation of a compound of formula Ib according to claim 1 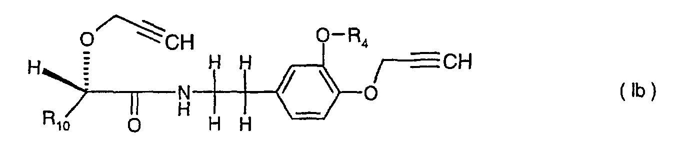 which comprises reacting a compound of formula 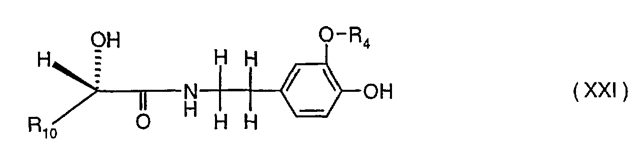 wherein R₄ and R₁₀ are as defined for formula I in claim 1, with a propargylating agent of formula XXII
Y-CH₂-≡CH (XXII)
wherein Y is a leaving group, e.g. bromine or chlorine or a tosyl, mesyl or trifluoromethylsulfonyl.

## Patentansprüche

1. Enantio-angereicherte Verbindungen der Formel I 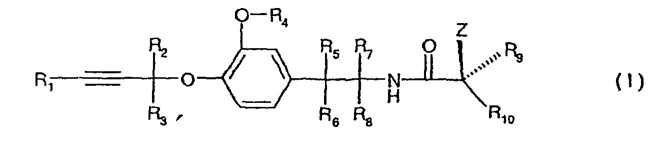 einschließlich der optischen Isomeren hiervon und Mischungen solcher Isomeren, worin
R₁ Wasserstoff, Alkyl, Cycloalkyl oder gegebenenfalls substituiertes Aryl darstellt,
R₂ und R₃ unabhängig voneinander Wasserstoff oder Alkyl darstellen,
R₄ Alkyl, Alkenyl oder Alkinyl darstellt,
R₅, R₆, R₇ und R₈ unabhängig voneinander Wasserstoff oder Alkyl darstellen,
R₉ Wasserstoff, gegebenenfalls substituiertes Alkyl, gegebenenfalls substituiertes Alkenyl oder gegebenenfalls substituiertes Alkinyl darstellt,
R₁₀ gegebenenfalls substituiertes Aryl oder gegebenenfalls substituiertes Heteroaryl darstellt, und
Z gegebenenfalls substituiertes Aryloxy, gegebenenfalls substituiertes Alkoxy, gegebenenfalls substituiertes Alkenyloxy oder gegebenenfalls substituiertes Alkinyloxy darstellt.

2. Verbindung gemäß Anspruch 1, worin die Enantio-angereicherte Form der Verbindung der Formel I das reine Enantiomer, wie dargestellt, in einer Menge von mindestens 70 % enthält.

3. Verbindung gemäß den Ansprüchen 1 oder 2, worin der Gehalt der Enantio-angereicherten Form das reine Enantiomer, wie dargestellt, in einer Menge von mindestens 80 %, bevorzugt über 90 %, insbesondere über 95 % enthält.

4. Verbindung gemäß den Ansprüchen 1 bis 3, worin
R₁ Wasserstoff, Alkyl, Cycloalkyl, Phenyl oder Naphthyl; Phenyl und Naphthyl gegebenenfalls substituiert durch Substituenten, ausgewählt aus der Gruppe umfassend Alkyl, Alkenyl, Alkinyl, Cycloalkyl, Cycloalkyl-alkyl, Phenyl und Phenylalkyl, wobei alle diese Gruppen wiederum substituiert sein können durch ein oder mehrere Halogene; Alkoxy, Alkenyloxy, Alkinyloxy; Alkoxy-alkyl; Halogenalkoxy; Alkylthio; Halogenalkylthio; Alkylsulfonyl; Formyl; Alkanoyl; Hydroxy; Halogen; Cyano; Nitro; Amino; Alkylamino; Dialkylamino; Carboxyl; Alkoxycarbonyl; Alkenyloxycarbonyl; oder Alkinyloxycarbonyl darstellt; und
R₂ und R₃ unabhängig voneinander Wasserstoff oder C₁-C₄-Alkyl darstellen; und
R₄ C₁-C₈-Alkyl, C₂-C₈-Alkenyl oder C₂-C₈-Alkinyl darstellt; und
R₅, R₆, R₇ und R₈ unabhängig voneinander Wasserstoff oder C₁-C₄-Alkyl darstellen; und
R₉ Wasserstoff, C₁-C₄-Alkyl, C₁-C₄-Halogenalkyl, C₃-C₄-Alkenyl oder C₃-C₄-Alkinyl darstellt; und
R₁₀ Aryl oder Heteroaryl, jedes gegebenenfalls substituiert mit Substituenten, ausgewählt aus der Gruppe umfassend Alkyl, Alkenyl, Alkinyl, Cycloalkyl, Cycloalkyl-alkyl, Phenyl und Phenylalkyl, worin alle diese Gruppen mit einem oder mehreren Substituenten substituiert sein können, ausgewählt aus der Gruppe umfassend Halogen; Alkoxy, Alkenyloxy, Alkinyloxy; Alkoxy-alkyl; Halogenalkoxy; Alkylthio, Halogenalkylthio; Alkylsulfonyl; Formyl; Alkanoyl; Hydroxy; Cyano; Nitro; Amino; Alkylamino; Dialkylamino; Carboxyl; Alkoxycarbonyl; Alkenyloxycarbonyl und Alkinyloxycarbonyl darstellt; und
Z gegebenenfalls substituiertes Aryloxy darstellt, worin das Aryl gegebenenfalls substituiert sein kann durch einen oder mehrere Substituenten, ausgewählt aus der Gruppe umfassend Halogen, C₁-C₈-Alkoxy, C₂-C₈-Alkenyloxy, C₂-C₈-Alkinyloxy, C₁-C₈-Alkoxy-C₁-C₈-alkyl, C₁-C₈-Halogenalkoxy, C₁-C₈-Alkylthio, C₁-C₈-Halogenalkylthio, C₁-C₈-Alkylsulfonyl, Formyl, C₂-C₈-Alkanoyl, Hydroxy, Halogen, Cyano, Nitro, Amino, C₁-C₈-Alkylamino, Di-C₁-C₈-Alkylamino, Carboxyl und C₁-C₈-Alkoxycarbonyl; oder gegebenenfalls substituiertes C₁-C₈-Alkoxy, gegebenenfalls substituiertes C₂-C₈-Alkenyloxy oder gegebenenfalls substituiertes C₂-C₈-Alkinyloxy darstellt, worin jede Alkyl-, Alkenyl- oder Alkinylgruppe einen oder mehrere Substituenten tragen kann, ausgewählt aus der Gruppe umfassend Halogen, C₁-C₄-Alkyl, C₁-C₄-Alkoxy, C₁-C₄-Alkylthio, C₃-C₆-Cycloalkyl, Nitro, Cyano, Hydroxy, Phenyl, Mercapto, C₁-C₄-Alkylcarbonyl und C₁-C₄-Alkoxycarbonyl.

5. Verbindung gemäß den Ansprüchen 1 bis 4, worin R₁ Wasserstoff, C₁-C₈-Alkyl, C₃-C₈-Cycloalkyl, Phenyl oder Naphthyl darstellt; Phenyl und Naphthyl gegebenenfalls substituiert sind durch einen bis drei Substituenten, ausgewählt aus der Gruppe umfassend C₁-C₈-Alkyl, C₂-C₈-Alkenyl, C₂-C₈-Alkinyl, C₁-C₈-Halogenalkyl, C₁-C₈-Alkoxy, C₁-C₈-Halogenalkoxy, C₁-C₈-Alkylthio, C₁-C₈-Halogenalkylthio, C₁-C₈-Alkylsulfonyl, Halogen, Cyano, Nitro und C₁-C₈-Alkoxycarbonyl; und R₂ und R₃ Wasserstoff darstellen; und R₄ C₁-C₆-Alkyl darstellt; und R₅, R₆ und R₇ Wasserstoff darstellen und R₈ Wasserstoff, Methyl oder Ethyl darstellt; und R₉ Wasserstoff oder C₁-C₄-Alkyl darstellt; und R₁₀ Phenyl, Naphthyl oder Biphenyl, jedes gegebenenfalls substituiert durch einen bis drei Substituenten, ausgewählt aus der Gruppe umfassend C₁-C₈-Alkyl, C₂-C₈-Alkenyl, C₂-C₈-Alkinyl, C₁-C₈-Halogenalkyl, C₁-C₈-Alkoxy, C₁-C₈-Halogenalkoxy, C₁-C₈-Alkylthio, C₁-C₈-Halogenalkylthio, C₁-C₈-Alkylsulfonyl, Halogen, Cyano, Nitro und C₁-C₈-Alkoxycarbonyl darstellt; und Z C₁-C₈-Alkoxy, C₂-C₈-Alkenyloxy, C₁-C₈-Alkinyloxy, C₁-C₈-Alkoxy-C₁-C₈-alkoxy, C₂-C₈-Alkenyloxy-C₁-C₈alkoxy, C₂-C₈-Alkinyloxy-C₁-C₈-alkoxy, C₁-C₈-Halogenalkoxy oder C₃-C₈-Cycloalkyl-C₁-C₈-alkoxy darstellt.

6. Verbindung der Formel I gemäß den Ansprüchen 1 bis 5, worin R₁ Wasserstoff, C₁-C₈-Alkyl oder Phenyl, gegebenenfalls substituiert durch einen bis drei Substituenten, ausgewählt aus der Gruppe umfassend C₁-C₈-Alkyl, C₁-C₈-Halogenalkyl, C₁-C₈-Alkoxy, C₁-C₈-Halogenalkoxy, C₁-C₈-Alkylthio, C₁-C₈-Halogenalkylthio, Halogen, Cyano, Nitro und C₁-C₈-Alkoxycarbonyl darstellt; und R₂ und R₃ Wasserstoff darstellen; und R₄ C₁-C₄-Alkyl darstellt, und R₅, R₆, R₇ Wasserstoff darstellen und R₈ Wasserstoff oder Methyl darstellt; und R₉ Wasserstoff darstellt; und R₁₀ Phenyl, Naphthyl, 1,3-Biphenyl oder 1,4-Biphenyl darstellt, jedes gegebenenfalls substituiert durch einen bis drei Substituenten, ausgewählt aus der Gruppe umfassend C₁-C₈-Alkyl, C₁-C₈-Halogenalkyl, C₁-C₈-Alkoxy, C₁-C₈-Halogenalkoxy, C₁-C₈-Alkylthio, C₁-C₈-Halogenalkylthio, Halogen, Cyano, Nitro und C₁-C₈-Alkoxycarbonyl; und Z C₁-C₈-Alkoxy, C₂-C₈-Alkenyloxy, C₂-C₈-Alkinyloxy oder C₁-C₄-Alkoxy-C₁-C₂-alkoxy darstellt.

7. Verbindung der Formel I gemäß den Ansprüchen 1 bis 6, worin R₁ Wasserstoff, C₁-C₈-Alkyl oder C₃-C₈-Cycloalkyl darstellt; und R₂, R₃, R₅, R₆, R₇, R₈ und R₉ Wasserstoff darstellen, und R₄ Methyl oder Ethyl darstellt; und R₁₀ Phenyl, Naphthyl, 1,3-Biphenyl oder 1,4-Biphenyl darstellt, jedes gegebenenfalls substituiert durch einen bis drei Substituenten, ausgewählt aus der Gruppe umfassend C₁-C₈-Alkyl, C₁-C₈-Halogenalkyl, C₁-C₈-Alkoxy, C₁-C₈-Halogenalkoxy, C₁-C₈-Alkylthio, C₁-C₈-Halogenalkylthio, Halogen, Cyano, Nitro und C₁-C₈-Alkoxycarbonyl.

8. Verbindung der Formel I gemäß Anspruch 1, ausgewählt aus der Gruppe umfassend
(S)-2-(4-Brom-phenyl)-2-methoxy-N-[2-(3-methoxy-4-prop-2-inyloxy-phenyl)-ethyl]-acetamid,
(S)-2-(4-Chlor-phenyl)-2-methoxy-N-[2-(3-methoxy-4-prop-2-inyloxy-phenyl)-ethyl]-acetamid,
(S)-2-(3,4-Dichlorphenyl)-2-methoxy-N-[2-(3-methoxy-4-prop-2-inyloxy-phenyl)-ethyl]-acetamid,
(S)-2-(4-Bromphenyl)-2-ethoxy-N-[2-(3-methoxy-4-prop-2-inyloxy-phenyl)-ethyl]-acetamid,
(S)-2-(4-Chlorphenyl)-2-ethoxy-N-[2-(3-methoxy-4-prop-2-inyloxy-phenyl)-ethyl]-acetamid,
(S)-2-(3,4-Dichlorphenyl)-2-ethoxy-N-[2-(3-methoxy-4-prop-2-inyloxy-phenyl)-ethyl]-acetamid,
(S)-2-(4-Bromphenyl)-N-[2-(3-methoxy-4-prop-2-inyloxy-phenyl)-ethyl]-2-propoxyacetamid,
(S)-2-(4-Chlorphenyl)-N-[2-(3-methoxy-4-prop-2-inylooy-phenyl)-ethyl]-2-propoxyacetamid,
(S)-2-(3,4-Dichlorphenyl)-N-[2-(3-methoxy-4-prop-2-inyloxy-phenyl)-ethyl]-2-propoxy-acetamid,
(S)-2-(4-Bromphenyl)-2-cyclopropylmethoxy-N-[2-(3-methoxy-4-prop-2-inyloxyphenyl)-ethyl]-acetamid,
(S)-2-(4-Chlorphenyl)-2-cyclopropylmethoxy-N-[2-(3-methoxy-4-prop-2-inyloxyphenyl)-ethyl]-acetamid,
(S)-2-Cyclopropylmethoxy-2-(3,4-Dichlorphenyl)-N-[2-(3-methoxy-4-prop-2-inyloxy-phenyl)-ethyl]-acetamid,
(S)-2-(4-Bromphenyl)-2-ethoxymethoxy-N-[2-(3-methoxy-4-prop-2-inyloxyphenyl)-ethyl]-acetamid,
(S)-2-(4-Chlorphenyl)-2-ethoxymethoxy-N-[2-(3-methoxy-4-prop-2-inyloxyphenyl)-ethyl]-acetamid,
(S)-2-(3,4-Dichlorphenyl)-2-ethoxymethoxy-N-[2-(3-methoxy-4-prop-2-inyloxyphenyl)-ethyl]-acetamid,
(S)-2-Allyloxy-2-(4-bromphenyl)-N-[2-(3-methoxy-4-prop-2-inyloxy-phenyl)-ethyl]-acetamid,
(S)-2-Allyloxy-2-(4-chlorphenyl)-N-[2-(3-methoxy-4-prop-2-inyloxy-phenyl)-ethyl]-acetamid,
(S)-2-Allyloxy-2-(3,4-dichlorphenyl)-N-[2-(3-methoxy-4-prop-2-inyloxy-phenyl)-ethyl]-acetamid,
(S)-2-(4-Bromphenyl)-2-(but-2-enyloxy)-N-[2-(3-methoxy-4-prop-2-inyloxyphenyl)-ethyl]-acetamid,
(S)-2-(But-2-enyloxy)-2-(4-chlorphenyl)-N-[2-(3-methoxy-4-prop-2-inyloxyphenyl)-ethyl]-acetamid,
(S)-2-(But-2-enyloxy)-2-(3,4-dichlorphenyl)-N-[2-(3-methoxy-4-prop-2-inyloxyphenyl)-ethyl]-acetamid,
(S)-2-(4-Bromphenyl)-N-[2-(3-methoxy-4-prop-2-inyloxy-phenyl)-ethyl]-2-prop-2-inyloxy-acetamid,
(S)-2-(4-Chlorphenyl)-N-[2-(3-methoxy-4-prop-2-inyloxy-phenyl)-ethyl]-2-prop-2-inyloxy-acetamid,
(S)-2-(3,4-Dichlorphenyl)-N-[2-(3-methoxy-4-prop-2-inyloxy-phenyl)-ethyl]-2-prop-2-inyloxy-acetamid,
(S)-2-Biphenyl-4-yl-N-[2-(3-methoxy-4-prop-2-inyloxy-phenyl)-ethyl]-2-prop-2-inyloxy-acetamid,
(S)-N-[2-(3-Methoxy-4-prop-2-inyloxy-phenyl)-ethyl]-2-naphthalin-2-yl-2-prop-2-inyloxy-acetamid,
(S)-N-[2-(3-Methoxy-4-prop-2-inyloxy-phenyl)-ethyl]-2-prop-2-inyloxy-2-p-tolylacetamid,
2-(4-Ethylphenyl)-N-[2-(3-methoxy-4-prop-2-inyloxy-phenyl)-ethyl]-2-prop-2-inyloxy-acetamid,
(S)-N-[2-(3-Methoxy-4-prop-2-inyloxy-phenyl)-ethyl]-2-prop-2-inyloxy-2-(4-trifluormethyl-phenyl)-acetamid,
(S)-2-(4-Bromphenyl)-2-but-2-inyloxy-N-[2-(3-methoxy-4-prop-2-inyloxy-phenyl)-ethyl]-acetamid,
(S)-2-But-2-inyloxy-2-(4-chlorphenyl)-N-[2-(3-methoxy-4-prop-2-inyloxy-phenyl)-ethyl]-acetamid,
(S)-2-But-2-inyloxy-2-(3,4-dichlorphenyl)-N-[2-(3-methoxy-4-prop-2-inyloxyphenyl)-ethyl]-acetamid,
(S)-2-(4-Bromphenyl)-N-[2-(3-methoxy-4-pent-2-inyloxy-phenyl)-ethyl]-2-prop-2-inyloxy-acetamid,
(S)-2-(4-Chlorphenyl)-N-[2-(3-methoxy-4-pent-2-inyloxy-phenyl)-ethyl]-2-prop-2-inyloxy-acetamid,
(S)-2-(3,4-Dichlorphenyl)-N-[2-(3-methoxy-4-pent-2-inyloxy-phenyl)-ethyl]-2-prop-2-inyloxy-acetamid,
(S)-2-(4-Fluorphenyl)-N-[2-(3-methoxy-4-prop-2-inyloxy-phenyl)-ethyl]-2-prop-2-inyloxy-acetamid,
(S)-2-(3,4-Difluorphenyl)-N-[2-(3-methoxy-4-prop-2-inyloxy-phenyl)-ethyl]-2-prop-2-inyloxy-acetamid,
(S)-2-(4-Chlor-3-fluorphenyl)-N-[2-(3-methoxy-4-prop-2-inyloxy-phenyl)-ethyl]-2-prop-2-inyloxy-acetamid, und
(S)-2-(3-Chlor-4-fluorphenyl)-N-[2-(3-methoxy-4-prop-2-inyloxy-phenyl)-ethyl]-2-prop-2-inyloxy-acetamid.

9. Verfahren zur Herstellung einer Verbindung der Formel I gemäß Anspruch 1, das umfasst die Reaktion
a) des Phenols der Formel IV 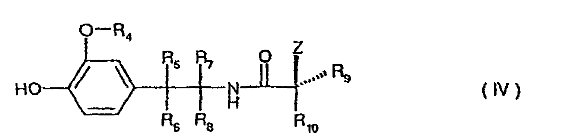 worin R₄, R₅, R₆, R₇, R₈, R₉, R₁₀ und Z wie für Formel I definiert sind, mit einer Verbindung der Formel V 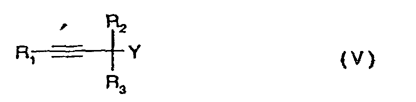 worin R₁, R₂ und R₃ wie für Formel I definiert sind und worin Y eine Abgangsgruppe darstellt; oder
b) der Säure der Formel II 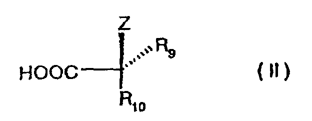 mit einem Amin der Formel VI 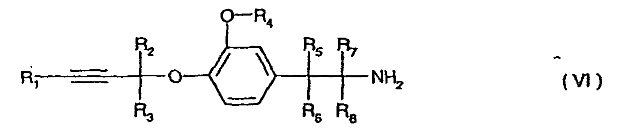 worin R₁, R₂, R₃, R₄, R₅, R₆, R₇, R₈, R₉ und R₁₀ wie für Formel I definiert sind.

10. Zusammensetzung zur Bekämpfung und zum Schutz gegenüber phytopathogenen Mikroorganismen, umfassend eine Verbindung der Formel I gemäß Anspruch 1 als Wirkstoff gemeinsam mit einem geeigneten Träger.

11. Zusammensetzung gemäß Anspruch 10, die zumindest eine zusätzliche fungizid wirksame Verbindung, bevorzugt ausgewählt aus der Gruppe bestehend aus Cymoxanil, Trifloxystrobin, Azoxystrobin, Picoxystrobin, Chlorothalonil, Metalaxyl, Metalaxyl-M, Pyraclostrobin (BAS500F), Dimethomorph, Fosetyl-Al, Kupfersalze, Acibenzolar-S-methyl, Fludioxonil, Mancozeb, Folpet, Fluazinam, Iprovalicarb, Zoxamid und (S)-2-(Methylsulfonyl-amino)-3-methyl-buttersäure N-[2-{3-Methoxy-4-(3-(4-chlorphenyl)- prop-2-in-1-yloxy)-phenyl}-ethyl]-amid umfasst.

12. Verwendung einer Verbindung der Formel I gemäß Anspruch 1, zum Schutz von Pflanzen gegen den Befall durch phytopathogene Mikroorganismen.

13. Methode zur Bekämpfung und Vorbeugung eines Befalls von Nutzpflanzen durch phytopathogene Mikroorganismen, die die Aufbringung einer Verbindung der Formel I gemäß Anspruch 1 als Wirkstoff auf die Pflanze, Teile der Pflanze oder den Standort hiervon umfasst.

14. Methode gemäß Anspruch 13, wobei die phytopathogenen Mikroorganismen Pilz-Organismen darstellen.

15. Verfahren zur Herstellung einer Verbindung der Formel Ia gemäß Anspruch 1 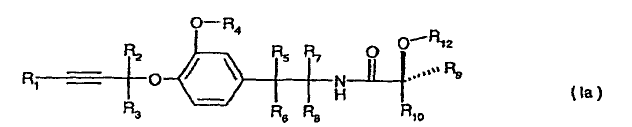 worin R₁, R₂, R₃, R₄, R₅, R₆, R₇, R₈, R₉ und Rio wie für Formel I in Anspruch 1 definiert sind, und R₁₂ Alkyl, Alkenyl oder Alkinyl darstellt, wobei das Verfahren umfasst die Reaktion einer Verbindung der Formel XIII 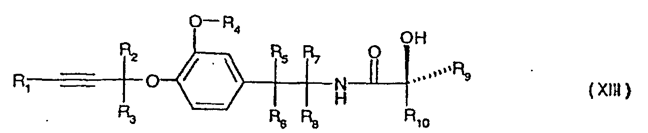 mit einer Verbindung der Formel XIV
Y-R₁₂ (XIV)
worin R₁₂ Alkyl, Alkenyl oder Alkinyl darstellt, und Y eine Abgangsgruppe darstellt, z.B. Brom oder Chlor oder ein Tosyl, Mesyl oder Trifluormethylsulfonyl.

16. Verfahren zur Herstellung einer Verbindung der Formel Ib gemäß Anspruch 1 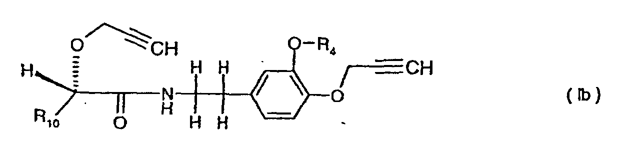 das die Reaktion einer Verbindung der Formel 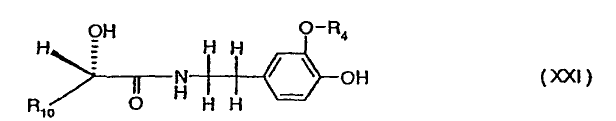 umfasst, worin R₄ und R₁₀ wie für Formel I in Anspruch 1 definiert sind, mit einem Propargylierungsmittel der Formel XXII
Y-CH₂-≡CH (XXII)
worin Y eine Abgangsgruppe darstellt, z.B. Brom oder Chlor oder ein Tosyl, Mesyl oder Trifluormethylsulfonyl.

## Revendications

1. Composés énantiomériquement enrichis de formule I 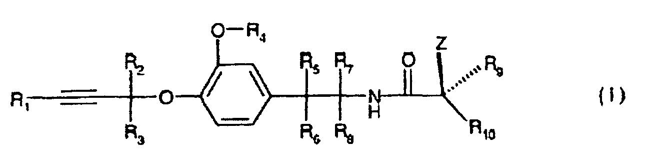 y compris leurs isomères optiques et les mélanges de tels isomères,
dans laquelle
R₁ représente un atome d'hydrogène ou un groupe alkyle, cycloalkyle ou aryle éventuellement substitué,
R₂ et R₃ représentent chacun indépendamment un atome d'hydrogène ou un groupe alkyle,
R₄ représente un groupe alkyle, alcényle ou alcynyle,
R₅, R₆, R₇, et R₈ représentent chacun indépendamment un atome d'hydrogène ou un groupe alkyle,
R₉ représente un atome d'hydrogène, un groupe alkyle éventuellement substitué, un groupe alcényle éventuellement substitué ou un groupe alcynyle éventuellement substitué,
R₁₀ représente un groupe aryle éventuellement substitué ou un groupe hétéroaryle éventuellement substitué, et
Z représente un groupe aryloxy éventuellement substitué, un groupe alcoxy, éventuellement substitué, un groupe alcényloxy éventuellement substitué ou un groupe alcynyloxy éventuellement substitué.

2. Composé selon la revendication 1, dans lequel la forme énantiomériquement enrichie du composé de formule I contient l'énantiomère pur représenté en une quantité d'au moins 70%.

3. Composé selon les revendications 1 ou 2, dans lequel la forme énantiomériquement enrichie contient l'énantiomère pur représenté en une quantité d'au moins 80%, de préférence supérieure à 90%, notamment supérieure à 95%.

4. Composé selon l'une quelconque des revendications 1 à 3, dans lequel R₁ représente un atome d'hydrogène ou un groupe alkyle, cycloalkyle, phényle ou naphtyle ; les groupes phényle et naphtyle étant éventuellement substitués par des substituants choisis parmi les groupes alkyle, alcényle, alcynyle, cycloalkyle, cycloalkylalkyle, phényle et phénylalkyle, tous ces groupes pouvant être à leur tour substitués par un ou plusieurs atomes halogènes ; ou les groupes alcoxy ; alcényloxy ; alcynyloxy ; alcoxyalkyle ; halogénoalcoxy ; alkylthio ; halogénoalkylthio ; alkylsulfonyle ; formyle ; alcanoyle ; hydroxyle ; cyano ; nitro ; amino ; alkylamino ; dialkylamino ; carboxyle ; alcoxycarbonyle ; alcényloxycarbonyle ; ou alcynyloxycarbonyle ; et
R₂ et R₃ représentent indépendamment l'un de l'autre un atome d'hydrogène ou un groupe alkyle en C₁-C₄ ; et
R₄ représente un groupe alkyle en C₁-C₈, un groupe alcényle en C₂-C₈, ou un groupe alcynyle en C₂-C₈ ; et
R₅, R₆, R₇ et R₈ représentent indépendamment les uns des autres, un atome d'hydrogène ou un groupe alkyle en C₁-C₄ ; et
R₉ représente un atome d'hydrogène, un groupe alkyle en C₁-C₄, un groupe halogénoalkyle en C₁-C₄, un groupe alcényle en C₃-C₄, ou un groupe alcynyle en C₃-C₄ ; et
R₁₀ représente un groupe aryle ou hétéroaryle, chacun étant éventuellement substitué par des substituants choisis parmi les groupes alkyle, alcényle, alcynyle, cycloalkyle, cycloalkylalkyle, phényle et phénylalkyle, tous ces groupes pouvant être substitués par un ou plusieurs substituants choisis dans le groupe comprenant un atome d'halogène ; les groupes alcoxy, alcényloxy, alcynyloxy ; alcoxyalkyle ; halogénoalcoxy ; alkylthio ; halogénoalkylthio ; alkylsulfonyle ; formyle ; alcanoyle ; hydroxyle ; cyano ; nitro ; amino ; alkylamino ; dialkylamino ; carboxyle ; alcoxycarbonyle ; alcényloxycarbonyle et alcynyloxycarbonyle ; et
Z représente un groupe aryloxy éventuellement substitué, dans lequel le groupe aryle peut être éventuellement substitué par un ou plusieurs substituants choisis dans le groupe comprenant un atome d'halogène, les groupes alcoxy en C₁-C₈, alcényloxy en C₂-C₈, alcynyloxy en C₂-C₈, (alcoxy en C₁-C₈)(alkyle en C₁-C₈), halogénoalcoxy en C₁-C₈, alkylthio en C₁-C₈, halogénoalkylthio en C₁-C₈, alkylsulfonyle en C₁-C₈, formyle, alcanoyle en C₂-C₈, hydroxyle, cyano, nitro, amino, alkylamino en C₁-C₈, dialkylamino en C₁-C₈, carboxyle et alcoxycarbonyle en C₁-C₈ ; ou Z représente un groupe alcoxy en C₁-C₈ éventuellement substitué, un groupe alcényloxy en C₂-C₈ éventuellement substitué ou un groupe alcynyloxy en C₂-C₈ éventuellement substitué, dans lequel chaque groupe alkyle, alcényle ou alcynyle peut porter un ou plusieurs substituants choisis dans le groupe comprenant un atome d'halogène, les groupes alkyle en C₁-C₄, alcoxy en C₁-C₄, alkylthio en C₁-C₄, cycloalkyle en C₃-C₆, nitro, cyano, hydroxyle, phényle, mercapto, alkylcarbonyle en C₁-C₄ et alcoxycarbonyle en C₁-C₄.

5. Composé selon l'une quelconque des revendications 1 à 4, dans lequel R₁ représente un atome d'hydrogène ou un groupe alkyle en C₁-C₈, cycloalkyle en C₃-C₈, phényle ou naphtyle ; les groupes phényle et naphtyle étant éventuellement substitués par 1 à 3 substituants choisis parmi un atome d'halogène ou les groupes alkyle en C₁-C₈, alcényle en C₂-C₈, alcynyle en C₂-C₈, halogénoalkyle en C₁-C₈, alcoxy en C₁-C₈, halogénoalcoxy en C₁-C₈, alkylthio en C₁-C₈, halogénoalkylthio en C₁-C₈, alkylsulfonyle en C₁-C₈, cyano, nitro, alcoxycarbonyle en C₁-C₈ ; et
R₂ et R₃ représentent un atome d'hydrogène ; et
R₄ représente un groupe alkyle en C₁-C₆ ; et
R₅, R₆ et R₇ représentent un atome d'hydrogène ; et
R₈ représente un atome d'hydrogène, ou les groupes méthyle et éthyle ; et
R₉ représente un atome d'hydrogène ou un groupe alkyle en C₁-C₄ ; et
R₁₀ représente un groupe phényle, naphtyle ou biphényle, chacun étant éventuellement substitué par 1 à 3 substituants choisis dans le groupe comprenant un atome d'halogène ou les groupes alkyle en C₁-C₈, alcényle en C₂-C₈, alcynyle en C₂-C₈, halogénoalkyle en C₁-C₈, alcoxy en C₁-C₈, halogénoalcoxy en C₁-C₈, alkylthio en C₁-C₈, halogénoalkylthio en C₁-C₈, alkylsulfonyle en C₁-C₈, cyano, nitro et alcoxycarbonyle en C₁-C₈ ; et
Z représente un groupe alcoxy en C₁-C₈, alcényloxy en C₂-C₈, alcynyloxy en C₂-C₈, (alcoxy en C₁-C₈)(alcoxy en C₁-C₈), (alcényloxy en C₂-C₈)(alcoxy en C₁-C₈), (alcynyloxy en C₂-C₈)(alcoxy en C₁-C₈), halogénoalcoxy en C₁-C₈ ou (cycloalkyle en C₃-C₈)(alcoxy en C₁-C₈).

6. Composé de formule I selon l'une quelconque des revendications 1 à 5,
dans lequel
R₁ représente un atome d'hydrogène, un groupe alkyle en C₁-C₈, ou un groupe phényle éventuellement substitué par 1 à 3 substituants choisis parmi un atome d'halogène ou les groupes alkyle en C₁-C₈, halogénoalkyle en C₁-C₈, alcoxy en C₁-C₈, halogénoalcoxy en C₁-C₈, alkylthio en C₁-C₈, halogénoalkylthio en C₁-C₈, cyano, nitro, alcoxycarbonyle en C₁-C₈ ; et
R₂ et R₃ représentent un atome d'hydrogène ; et
R₄ représente un groupe alkyle en C₁-C₄ ; et
R₅, R₆ et R₇ représentent un atome d' hydrogène ; et
R₈ représente un atome d'hydrogène, ou un groupe méthyle ; et
R₉ représente un atome d'hydrogène ; et
R₁₀ représente un groupe phényle, naphtyle, 1,3-biphényle ou 1,4-biphényle, chacun étant éventuellement substitué par 1 à 3 substituants choisis dans le groupe comprenant un atome d'halogène ou les groupes alkyle en C₁-C₈, halogénoalkyle en C₁-C₈, alcoxy en C₁-C₈, halogénoalcoxy en C₁-C₈, alkylthio en C₁-C₈, halogénoalkylthio en C₁-C₈, cyano, nitro et alcoxycarbonyle en C₁-C₈ ; et
Z représente un groupe alcoxy en C₁-C₈, alcényloxy en C₂-C₈, alcynyloxy en C₂-C₈ ou (alcoxy en C₁-C₄)(alcoxy en C₁-C₂).

7. Composé de formule I selon l'une quelconque des revendications 1 à 6, dans lequel
R₁ représente un atome d'hydrogène, un groupe alkyle en C₁-C₈ ou un groupe cycloalkyle en C₃-C₈ ; et
R₂, R₃, R₅, R₆, R₇, R₈ et R₉ représentent chacun un atome d'hydrogène ; et
R₄ représente un groupe méthyle ou un groupe éthyle ; et
R₁₀ représente un groupe phényle, naphtyle, 1,3-biphényle ou 1,4-biphényle, chacun étant éventuellement substitué par 1 à 3 substituants choisis dans le groupe comprenant un atome d'halogène ou les groupes alkyle en C₁-C₈, halogénoalkyle en C₁-C₈, alcoxy en C₁-C₈, halogénoalcoxy en C₁-C₈, alkylthio en C₁-C₈, halogénoalkylthio en C₁-C₈, cyano, nitro et alcoxycarbonyle en C₁-C₈.

8. Composé de formule I selon la revendication 1, représenté par un élément de l'ensemble formé par
le (S)-2-(4-bromo-phényl)-2-méthoxy-N-[2-(3-méthoxy-4-prop-2-ynyloxy-phényl)-éthyl]acétamide,
le (S)-2-(4-chloro-phényl)-2-méthoxy-N-[2-(3-méthoxy-4-prop-2-ynyloxy-phényl)-éthyl]acétamide,
le (S)-2-(3,4-dichloro-phényl)-2-méthoxy-N-[2-(3-méthoxy-4-prop-2-ynyloxy-phényl)-éthyl]acétamide,
le (S)-2-(4-bromo-phényl)-2-éthoxy-N-[2-(3-méthoxy-4-prop-2-ynyloxy-phényl)-éthyl]acétamide,
le (S)-2-(4-chloro-phényl)-2-éthoxy-N-[2-(3-méthoxy-4-prop-2-ynyloxy-phényl)-éthyl]acétamide,
le (S)-2-(3,4-dichloro-phényl)-2-éthoxy-N-[2-(3-méthoxy-4-prop-2-ynyloxy-phényl)-éthyl]acétamide,
le (S)-2-(4-bromo-phényl)-N-[2-(3-méthoxy-4-prop-2-ynyloxy-phényl)-éthyl]-2-propoxy-acétamide,
le (S)-2-(4-chloro-phényl)-N-[2-(3-méthoxy-4-prop-2-ynyloxy-phényl)-éthyl]-2-propoxy-acétamide,
le (S)-2-(3,4-dichloro-phényl)-N-[2-(3-méthoxy-4-prop-2-ynyloxy-phényl)-éthyl]-2-propoxy-acétamide,
le (S)-2-(4-bromo-phényl)-2-cyclopropylméthoxy-N-[2-(3-méthoxy-4-prop-2-ynyloxy-phényl)-éthyl]acétamide,
le (S)-2-(4-chloro-phényl)-2-cyclopropylméthoxy-N-[2-(3-méthoxy-4-prop-2-ynyloxy-phényl)-éthyl]acétamide,
le (S)-2-cyclopropylméthoxy-2-(3,4-dichloro-phényl)-N-[2-(3-méthoxy-4-prop-2-ynyloxy-phényl)-éthyl]acétamide,
le (S)-2-(4-bromo-phényl)-2-éthoxyméthoxy-N-[2-(3-méthoxy-4-prop-2-ynyloxy-phényl)-éthyl]acétamide,
le (S)-2-(4-chloro-phényl)-2-éthoxyméthoxy-N-[2-(3-méthoxy-4-prop-2-ynyloxy-phényl)-éthyl]acétamide,
le (S)-2-(3,4-dichloro-phényl)-2-éthoxyméthoxy-N-[2-(3-méthoxy-4-prop-2-ynyloxy-phényl)-éthyl]acétamide,
le (S)-2-allyloxy-2-(4-bromo-phényl)-N-[2-(3-méthoxy-4-prop-2-ynyloxy-phényl)-éthyl]acétamide,
le (S)-2-allyloxy-2-(4-chloro-phényl)-N-[2-(3-méthoxy-4-prop-2-ynyloxy-phényl)-éthyl]acétamide,
le (S)-2-allyloxy-2-(3,4-dichloro-phényl)-N-[2-(3-méthoxy-4-prop-2-ynyloxy-phényl)-éthyl]acétamide,
le (S)-2-(4-bromo-phényl)-2-(but-2-ényloxy)-N-[2-(3-méthoxy-4-prop-2-ynyloxy-phényl)-éthyl]acétamide,
le (S)-2-(but-2-ényloxy)-2-(4-chloro-phényl)-N-[2-(3-méthoxy-4-prop-2-ynyloxy-phényl)-éthyl]acétamide,
le (S)-2-(but-2-ényloxy)-2-(3,4-dichloro-phényl)-N-[2-(3-méthoxy-4-prop-2-ynyloxy-phényl)-éthyl]acétamide,
le (S)-2-(4-bromo-phényl)-N-[2-(3-méthoxy-4-prop-2-ynyloxy-phényl)-éthyl]-2-prop-2-ynyloxy-acétamide,
le (S)-2-(4-chloro-phényl)-N-[2-(3-méthoxy-4-prop-2-ynyloxy-phényl)-éthyl]-2-prop-2-ynyloxy-acétamide,
le (S)-2-(3,4-dichloro-phényl)-N-[2-(3-méthoxy-4-prop-2-ynyloxy-phényl)-éthyl]-2-prop-2-ynyloxy-acétamide,
le (S)-2-biphényl-4-yl-N-[2-(3-méthoxy-4-prop-2-ynyloxy-phényl)-éthyl]-2-prop-2-ynyloxy-acétamide,
le (S)-N-[2-(3-méthoxy-4-prop-2-ynyloxy-phényl)-éthyl]-2-naphtalén-2-yl-prop-2-ynyloxy-acétamide,
le (S)-N-[2-(3-méthoxy-4-prop-2-ynyloxy-phényl)-éthyl]-2-prop-2-ynyloxy-2-p-tolyl-acétamide,
le (S)-2-(4-éthyl-phényl)-N-[2-(3-méthoxy-4-prop-2-ynyloxy-phényl)-éthyl]-2-prop-2-ynyloxy-acétamide,
le (S)-N-[2-(3-méthoxy-4-prop-2-ynyloxy-phényl)-éthyl]-2-prop-2-ynyloxy-2-(4-trifluorométhyl-phényl)acétamide,
le (S)-2-(4-bromo-phényl)-2-but-2-ynyloxy-N-[2-(3-méthoxy-4-prop-2-ynyloxy-phényl)-éthyl]-acétamide,
le (S)-2-but-2-ynyloxy-2-(4-chloro-phényl)-N-[2-(3-méthoxy-4-prop-2-ynyloxy-phényl)-éthyl]-acétamide,
le (S)-2-but-2-ynyloxy-2-(3,4-dichloro-phényl)-N-[2-(3-méthoxy-4-prop-2-ynyloxy-phényl)-éthyl]-acétamide,
le (S)-2-(4-bromo-phényl)-N-[2-(3-méthoxy-4-pent-2-ynyloxy-phényl)-éthyl]-2-prop-2-ynyloxy-acétamide,
le (S)-2-(4-chloro-phényl)-N-[2-(3-méthoxy-4-pent-2-ynyloxy-phényl)-éthyl]-2-prop-2-ynyloxy-acétamide,
le (S)-2-(3,4-dichloro-phényl)-N-[2-(3-méthoxy-4-pent-2-ynyloxy-phényl)-éthyl]-2-prop-2-ynyloxy-acétamide,
le (S)-2-(4-fluoro-phényl)-N-[2-(3-méthoxy-4-prop-2-ynyloxy-phényl)-éthyl]-2-prop-2-ynyloxy-acétamide,
le (S)-2-(3,4-difluoro-phényl)-N-[2-(3-méthoxy-4-prop-2-ynyloxy-phényl)-éthyl]-2-prop-2-ynyloxy-acétamide,
le (S)-2-(4-chloro-3-fluoro-phényl)-N-[2-(3-méthoxy-4-prop-2-ynyloxy-phényl)-éthyl]-2-prop-2-ynyloxy-acétamide, et
le (S)-2-(3-chloro-4-fluoro-phényl)-N-[2-(3-méthoxy-4-prop-2-ynyloxy-phényl)-éthyl]-2-prop-2-ynyloxy-acétamide.

9. Procédé de préparation d'un composé de formule I selon la revendication 1, qui comprend la mise en réaction
a) du phénol de formule IV 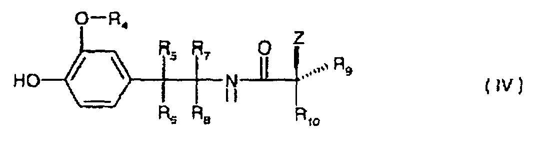 dans laquelle R₄, R₅, R₆, R₇, R₈, R₉, R₁₀ et Z ont les significations indiquées pour la formule I, avec un composé de formule V 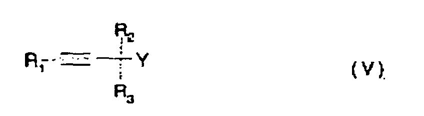 dans laquelle R₁, R₂ et R₃ ont les significations indiquées pour la formule I et dans laquelle Y représente un groupe partant ; ou
b) l'acide de formule II 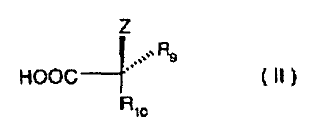 avec l'amine de formule VI 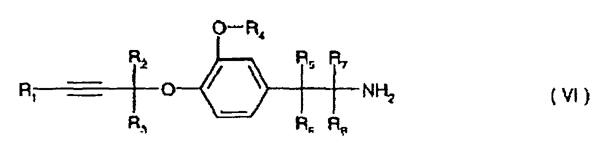 dans lesquelles R₁, R₂, R₃, R₄, R₅, R₆, R₇, R₈, R₉ et R₁₀ ont les significations indiquées pour la formule I.

10. Composition destinée au contrôle et à la protection contre des microorganismes phytopathogènes, comprenant un composé de formule I selon la revendication 1, en tant que principe actif, conjointement avec un véhicule approprié.

11. Composition selon la revendication 10, qui comprend au moins un composé fongicide supplémentaire, de préférence représenté par un élément de l'ensemble formé par le cymoxanil, la trifloxystrobine, l'azoxystrobine, la picoxystrobine, le chlorothalonil, le métalaxyl, le métalaxyl-M, la pyraclostrobine (BAS500F), le diméthomorphe, le fosétyl-Al, les sels de cuivre, l'acibenzolar-S-méthyl, le fludioxonil, le mancozèbe, le folpel, le fluazinam, l'iprovalicarbe, la zoxamide et le N-[2-(3-méthoxy-4-(3-(4-chlorophényl)-2-propyn-1-yloxy)-phényl)-éthyl]-amide de l'acide (S)-2-(méthylsulfonyl-amino)-3-méthylbutyrique.

12. Utilisation d'un composé de formule I selon la revendication 1, en vue de protéger les plantes contre l'infestation par des microorganismes phytopathogènes.

13. Procédé permettant de contrôler et de prévenir l'infestation des plantes cultivées par des microorganismes phytopathogènes, qui comprend l'application d'un composé de formule I selon la revendication 1, en tant que principe actif, à des plantes, à des parties de plantes ou à leur emplacement.

14. Procédé selon la revendication 13, dans lequel les microorganismes phytopathogènes sont les organismes fongiques.

15. Procédé de préparation d'un composé de formule Ia selon la revendication 1 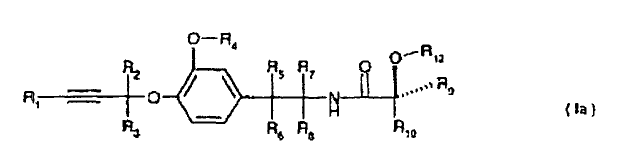 dans laquelle R₁, R₂, R₃, R₄, R₅, R₆, R₇, R₈, R₉ et R₁₀ ont les significations indiquées pour la formule I dans la revendication 1, et R₁₂ représente un groupe alkyle, alcényle ou alcynyle, lequel procédé comprend la mise en réaction d'un composé de formule XIII 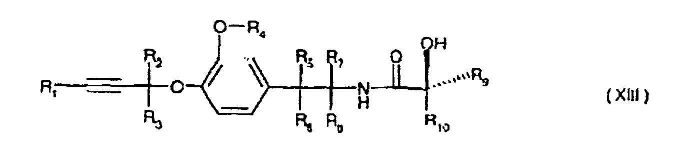 avec un composé de formule XIV
Y-R₁₂ (XIV)
dans laquelle R₁₂ représente un groupe alkyle, alcényle ou alcynyle, et Y représente un groupe partant, par exemple un atome de brome ou un atome de chlore ou un groupe tosyle, mésyle ou trifluorométhylsulfonyle.

16. Procédé de préparation d'un composé de formule Ib selon la revendication 1 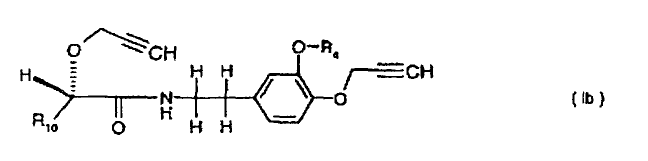 qui comprend la mise en réaction d'un composé de formule XXI 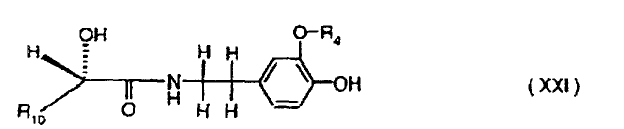 dans laquelle R₄ et R₁₀ ont les significations indiquées pour la formule I dans la revendication 1, avec un agent de propargylation de formule XXII
Y-CH₂-≡CH (XXII)
dans laquelle Y représente un groupe partant, par exemple un atome de brome ou un atome de chlore ou un groupe tosyle, mésyle ou trifluorométhylsulfonyle.
